# EUROPEAN PATENT APPLICATION

(11) **EP 1 026 142 A1**
(43) Date of publication of application: **09.08.2000**
(21) Application number: 98950384.2
(22) Date of filing: 26.10.1998
(51) Int. Cl.: C07C 43/225, C07C 25/18, C07C 69/74, C07C 69/76, C07D 319/06, C07D 309/06, C09K 19/10, C09K 19/20, C09K 19/30, C09K 19/34, C09K 19/42, G02F 1/13

(54) **2,3-DIFLUOROPHENYL DERIVATIVES HAVING NEGATIVE VALUE OF PERMITTIVITY ANISOTROPY, LIQUID-CRYSTAL COMPOSITION, AND LIQUID-CRYSTAL DISPLAY ELEMENT**

(30) Priority: 24.10.1997 JP 30991997
(71) Applicant: CHISSO CORPORATION, Osaka-shi, Osaka-fu 530-0005 (JP)
(72) Inventor: MIYAZAWA, Kazutoshi, Yachiyo-shi Chiba 276-0022 (JP); TAKEUCHI, Hiroyuki, Ichihara-shi Chiba 290-0022 (JP); YAGI, Yoshitaka, Chiba-shi Chiba 263-0032 (JP); TAKESHITA, Fusayuki, Sodegaura-shi Chiba 299-0261 (JP); NAKAGAWA, Etsuo, Ichihara-shi Chiba 290-0056 (JP)
(74) Representative: HOFFMANN - EITLE
(86) International application number: JP9804833
(87) International publication number: WO9921815

(57) **Abstract**

The invention is to provide liquid crystalline compounds having a negative and large dielectric anisotropy value and a small optical anisotropy value; liquid crystal compositions comprising the compound; and liquid crystal display devices fabricated by using the liquid crystal composition; the liquid crystalline compounds have 2,3-difluorophenyl moiety and are expressed by the general formula (1) 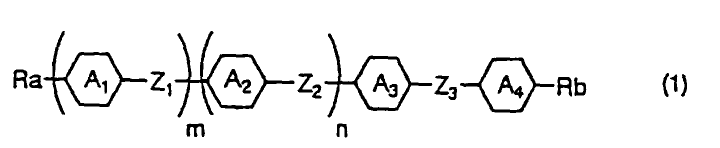 wherein Ra and Rb represent an alkyl group or alkoxy group having 1 to 10 carbon atoms, but any one or more methylene groups in at least one of the Ra and Rb are replaced by cyclopropane-1,2-diyl, -CF₂-, or -CFH-; ring A₁ to ring A₄ represent cyclohexane-1,4-diyl or 1,4-phenylene, but at least one of the ring A₃ and ring A₄ represents 2,3-difluoro-1,4-phenylene; Z₁, Z₂, and Z₃ represent single bond, -(CH₂)ₚ-, -CO₂-, -CF₂O-, or -CH₂O-; p is an integer of 2 to 4; and m and n are 0 or 1.

## Description

### TECHNICAL FIELD

The present invention relates to novel liquid crystalline compounds and liquid crystal compositions. More specifically, the invention relates to liquid crystalline compounds having, at a terminal position of the molecule, an alkyl group or alkoxy group in which fluorine atom is introduced, an alkyl group or alkoxy group in which cyclopropane-1,2-diyl group is introduced, or an alkyl group or alkoxy group in which both fluorine atom and cyclopropane-1,2-diyl group are introduced; to liquid crystal compositions comprising the compound; and to liquid crystal display devices fabricated by using the liquid crystal composition

### BACKGROUND ART

Liquid crystal display devices fabricated by using liquid crystalline compounds (the term "liquid crystalline compounds" is used in this specification as a generic name for the compounds which exhibit a liquid crystal phase and the compounds which do not exhibit a liquid crystal phase but are useful as a component of liquid crystal compositions) are widely being used for the display of computers, television sets, and the likes.

For the purpose of reducing the power consumption and decreasing the leakage of electromagnetic wave, liquid crystal compositions are required to lower their driving voltage. Driving voltage (threshold voltage) is known to be a function of dielectric anisotropy value and elastic constant according to the following equation (M. F. Leslie, Mol. Cryst. Liq. Cryst., 12, 57 (1970)):${\text{Vth= π(K/ε}}_{\text{0}} {\text{Δε)}}^{\text{1/2}}$ wherein Vth represents a threshold voltage, ε₀: a dielectric constant in vacuum, K: an elastic constant, and Δε: a dielectric anisotropy, respectively.

That is, it can be understood that in order to lower driving voltage, it is necessary 1) to increase dielectric anisotropy and 2) to reduce elastic constant.

It is generally considered to be difficult to adjust the value of the elastic constant of the compounds, and thus a means in which the dielectric anisotropy value is increased is principally adopted to lower the driving voltage. Accordingly, novel liquid crystalline compounds having a large dielectric anisotropy value are long-expected.

From some time ago, a characteristic of a narrow visual angle is considered to be a most serious problem to liquid crystal display devices, and thus various display modes have been proposed in recent years for the purpose of improving the narrow visual angle. In-plane switching (IPS) display devices proposed in 1995 greatly widened the visual angle compared with conventional display devices (Lecture on Liquid Crystals 2A07 (1995), ASIA DISPLAY '95, 557 (1995), and ASIA DISPLAY '95, 707 (1995)).

Also, in 1997, an attempt was reported in which a vertical alignment (VA) cell was used (SID 97 DIGEST, 845 (1997)), and the display devices of even this mode are considerably wide in visual angle compared with conventional display devices.

In either mode of IPS and VA, characteristics required of liquid crystal compositions are
1) a negative and large dielectric anisotropy value (Δε) for lowering driving voltage, and
2) a small optical anisotropy value (Δn) for keeping Δn·d (product of optical anisotropy value multiplied by cell thickness) at an optimum value.

However, compounds having simultaneously a negative and large dielectric anisotropy value and a small optical anisotropy value are heretofore unknown, and thus novel liquid crystalline compounds having such characteristics have been long-expected.

As a value which indicates the extent of the interaction between liquid crystal molecules and is inherent to the substance, order parameter is known. Dielectric anisotropy value and optical anisotropy value are regarded as functions of the order parameter (W. Maier and G. Meier, Z. Naturf. (a), 16, 262 (1961)), and the optical anisotropy value can be decreased by decreasing the order parameter. However, the optical anisotropy and dielectric anisotropy are in the relation of trade-off, and the decrease in the order parameter also decreases the dielectric anisotropy value.

Accordingly, novel compounds having such a peculiar property that only optical anisotropy value is decreased without causing a substantial decrease in dielectric anisotropy value have been much-awaited.

As compounds having a negative and large dielectric anisotropy value and a comparatively small optical anisotropy value, the compound of the following formula (13) is known (V. Reifffenrath et al., Liq. Cryst., 5 (1), 159 (1989)). It is reported that the dielectric anisotropy value (Δε) of this compound is -4.4 and optical anisotropy value (Δn) is 0.13. Whereas the compound (13) exhibits a large dielectric anisotropy value, its optical anisotropy value is large. Thus, it was unable to satisfy the value required for IPS mode or VA mode.

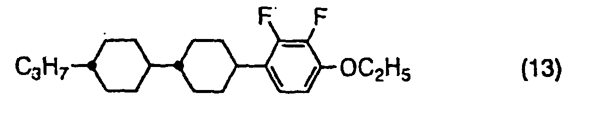

As compounds having a small optical anisotropy value, the compound in which cyano group is introduced and expressed by the formula (14) is also known (R. Eidenschink et al., Angew. Chem., 96, 151 (1984)). This compound has a large dielectric anisotropy value and a small optical anisotropy value together. However, it is extremely low in chemical stability, specific resistivity, and voltage holding ratio. Liquid crystal compositions for IPS mode or VA mode comprising the compound are considerably low in contrast. Thus, such compound was unsuitable as liquid crystalline compound to be used in liquid crystal compositions for such applications.

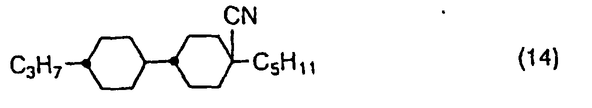

### DISCLOSURE OF THE INVENTION

In view of the characteristics required of liquid crystalline compounds and described above, an object of the present invention is to provide liquid crystalline compounds having a negative and extremely large dielectric anisotropy value and a small optical anisotropy value at the same time, to provide liquid crystal compositions comprising the compound, and to provide liquid crystal display devices fabricated by using the liquid crystal composition.

As a result of diligent research and development by the present inventors, it has now been found out that the liquid crystalline compounds expressed by the following general formula (1) have desired characteristics, leading to the completion of the present invention. 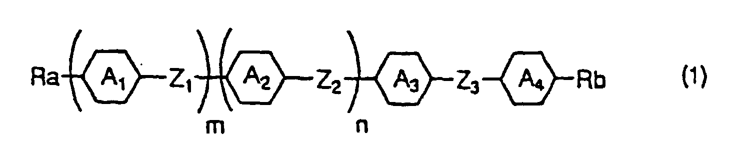 wherein Ra and Rb each independently represent a straight chain or branched alkyl group having 1 to 10 carbon atoms or a straight chain or branched alkoxy group having 1 to 10 carbon atoms in which groups any methylene group may be replaced by -O-, -CH=CH-, or -C≡C-, but there is not a case wherein -O-continues, provided that any one or more methylene groups in at least one of the Ra and Rb are replaced by cyclopropane-1,2-diyl, -CF₂-, or -CFH-; ring A₁ to ring A₄ each independently represent cyclohexane-1,4-diyl or 1,4-phenylene, but hydrogen atom on these rings may be replaced by a halogen atom and not-adjacent any methylene group in the cyclohexane ring may be replaced by -O-, provided that at least one of the ring A₃ and ring A₄ represents 2,3-difluoro-1,4-phenylene; Z₁, Z₂, and Z₃ each independently represent single bond, -(CH₂)ₚ-, -CO₂-, -CF₂O-, or -CH₂O- provided that p is an integer of 2 to 4; m and n are each independently 0 or 1; and any atom which constitutes the compound may be replaced by its isotope.

Among the liquid crystalline compounds expressed by the general formula (1), compounds which exhibit especially preferable characteristics are those expressed by one of the following general formulas (1-1) to (1-30):

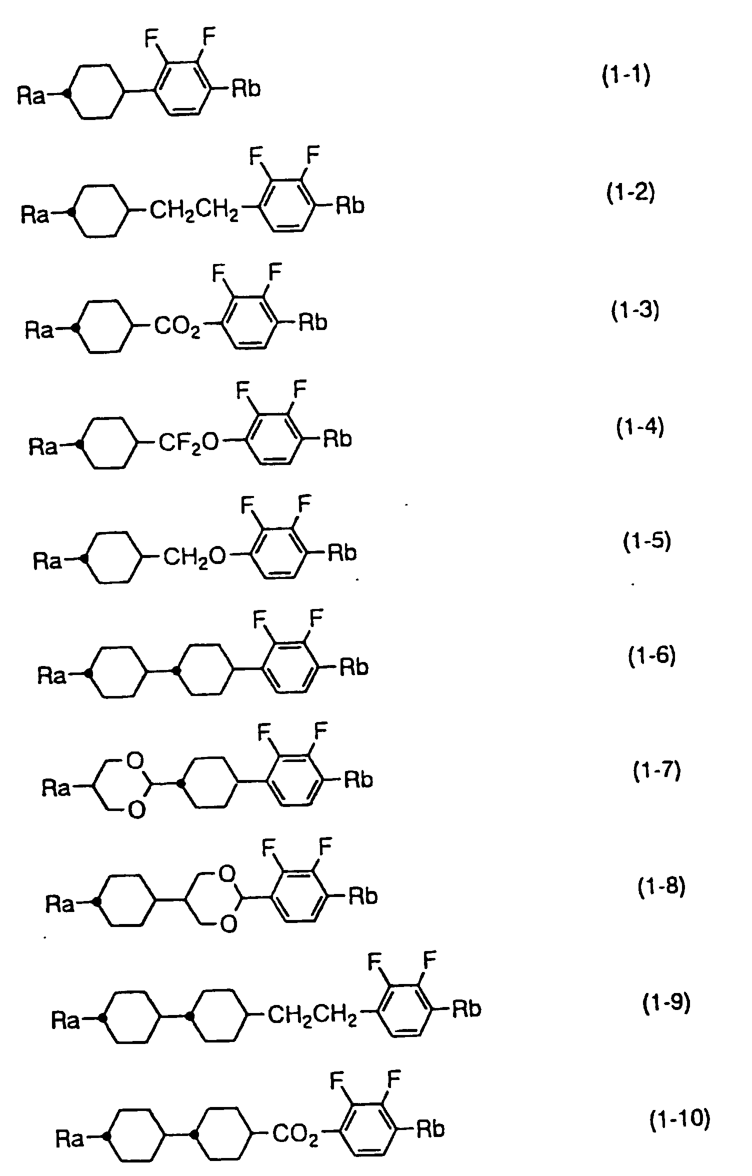

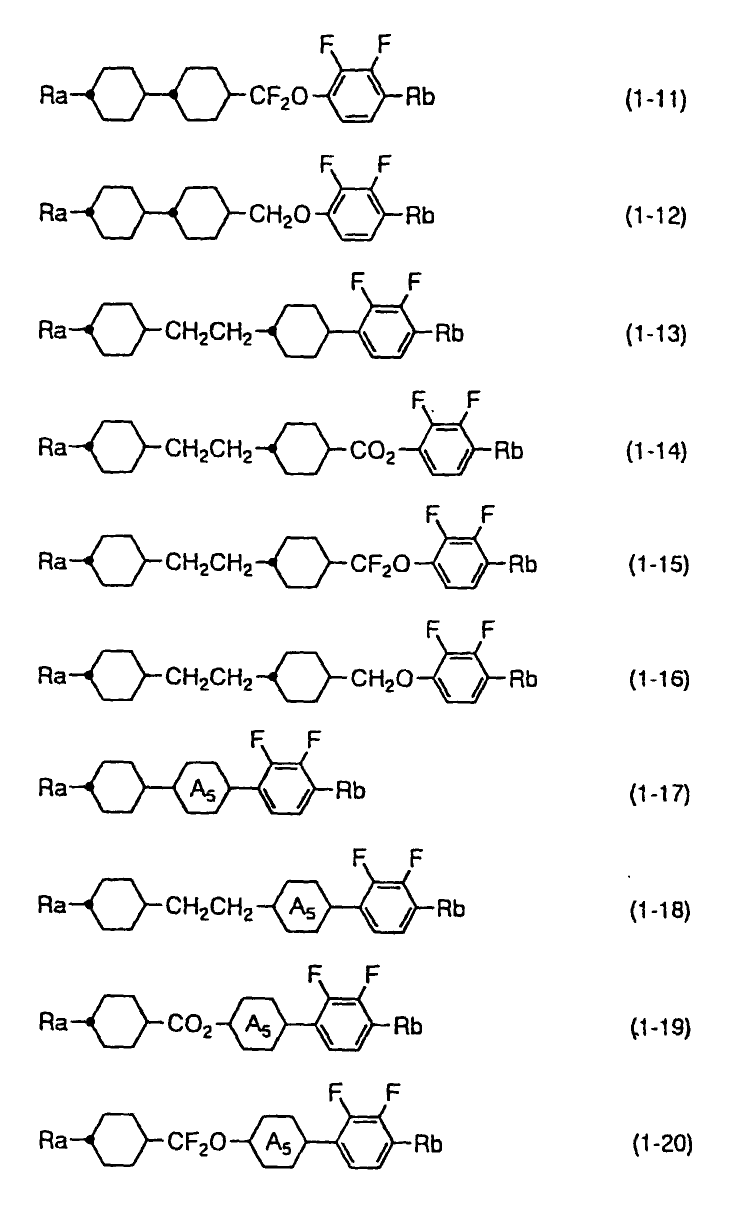

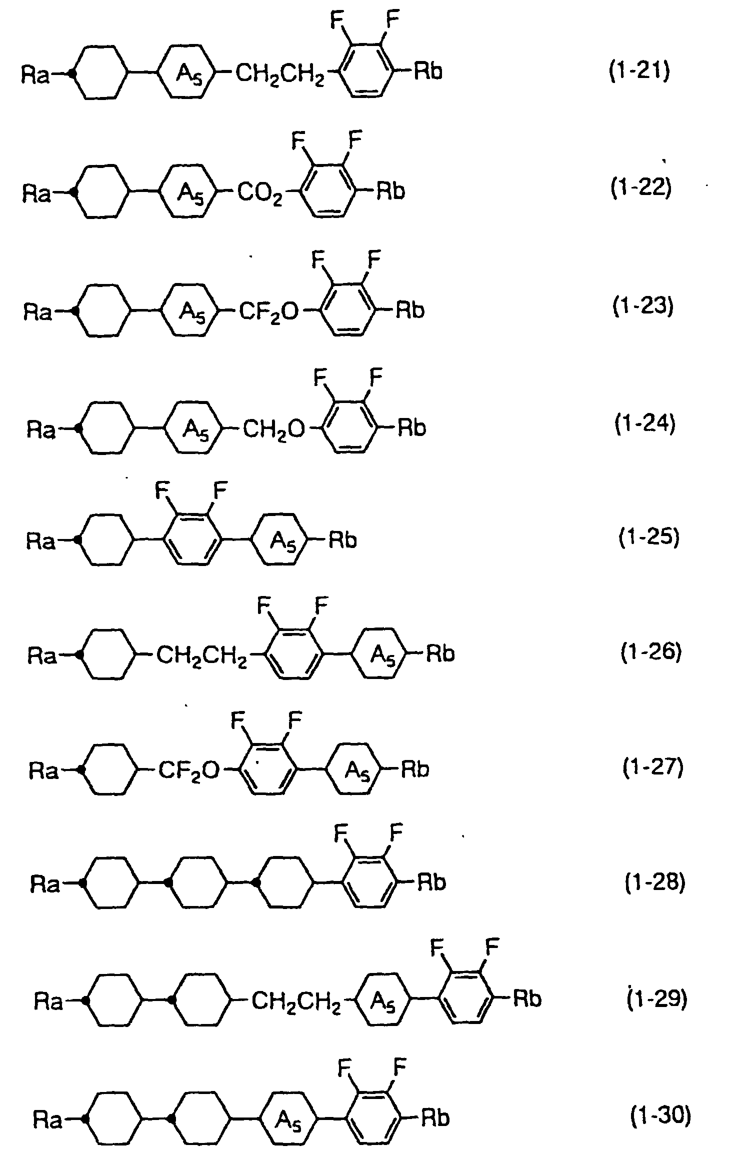

In the general formulas (1-17) to (1-27), (1-29), and (1-30) described above, ring A₅ represents any one of the following structures.

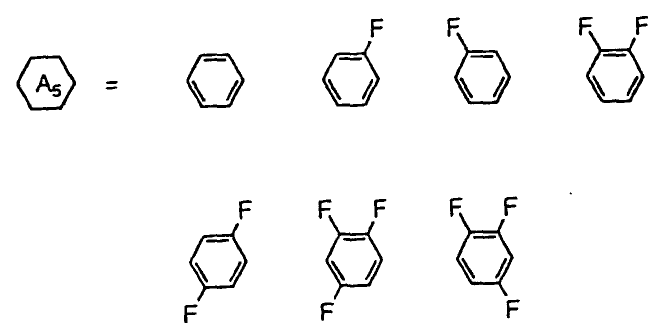

In the general formula (1), Ra and Rb are alkyl groups, alkoxy groups, alkoxyalkyl groups, alkoxyalkoxy groups, alkenyl groups, or alkynyl groups.

Compounds in which Ra and Rb are alkyl groups, alkoxy groups, alkoxyalkyl groups, or alkoxyalkoxy groups are chemically stable, and the compounds having alkenyl groups or alkynyl groups exhibit a slightly large optical anisotropy.

Besides, the compounds in which Ra and Rb are groups replaced by fluorine atom are large in dielectric anisotropy value, low in viscosity, and further, excellent in mutual solubility with other liquid crystal compounds.

Compounds expressed by the general formula (1) wherein Ra and Rb are groups replaced by cyclopropane-1,2-diyl groups are large in dielectric anisotropy value and particularly excellent in mutual solubility with other liquid crystal compounds.

Compound in which Ra and Rb are alkyl groups, alkoxy groups, or alkenyl groups are preferable since they have a low viscosity.

Further, when used in display devices for IPS mode or VA mode, the compounds in which Ra and Rb are alkyl groups or alkoxy groups are optimum, since a high chemical stability and a small optical anisotropy are required.

While Z₁, Z₂, and Z₃ are single bonds, -CH₂CH₂-, -(CH₂)₄-, -CO₂-, -CF₂O-, or -CH₂O-, compounds in which Z₁, Z₂, and Z₃ are single bonds or -CO₂- have a lower viscosity, the compounds in which Z₁ to Z₃ are -CH₂CH₂- exhibit a wider temperature range of nematic phase, and the compounds in which Z₁ to Z₃ are -CF₂O-exhibit an especially low viscosity.

Rings A₁ to A₄ are 2,3-difluoro-1,4-phenylene, cyclohexane-1,4-diyl, 1,3-dioxane-2,5-diyl, tetrahydropyran-2,5-diyl, or 1,4-phenylene, but at least one of ring A₃ and ring A₄ is 2,3-difluoro-1,4-phenylene.

When compounds of a low viscosity are produced, cyclohexane-1,4-diyl is optimum for ring A₁ to ring A₄. Compounds in which 1,3-dioxane-2,5-diyl is introduced as ring A₁ to ring A₄ are preferable since elastic constant K decreases and thus driving voltage of liquid crystal display devices of TN mode (including IPS and VA modes) lowers.

Besides, when it is desired to provide compounds of a small optical anisotropy value in particular, the compounds had better have a small number of 1,4-phenylene ring.

Compounds of the present invention expressed by the general formula (1) can be produced by using known procedures in organic synthetic chemistry in a proper combination. The known procedures in organic chemistry can be found by consulting books such as Organic Synthesis, Organic Reactions, and Shin-Jikken Kagaku Kouza (Course of New Chemical Experiment). Their typical examples are shown below.

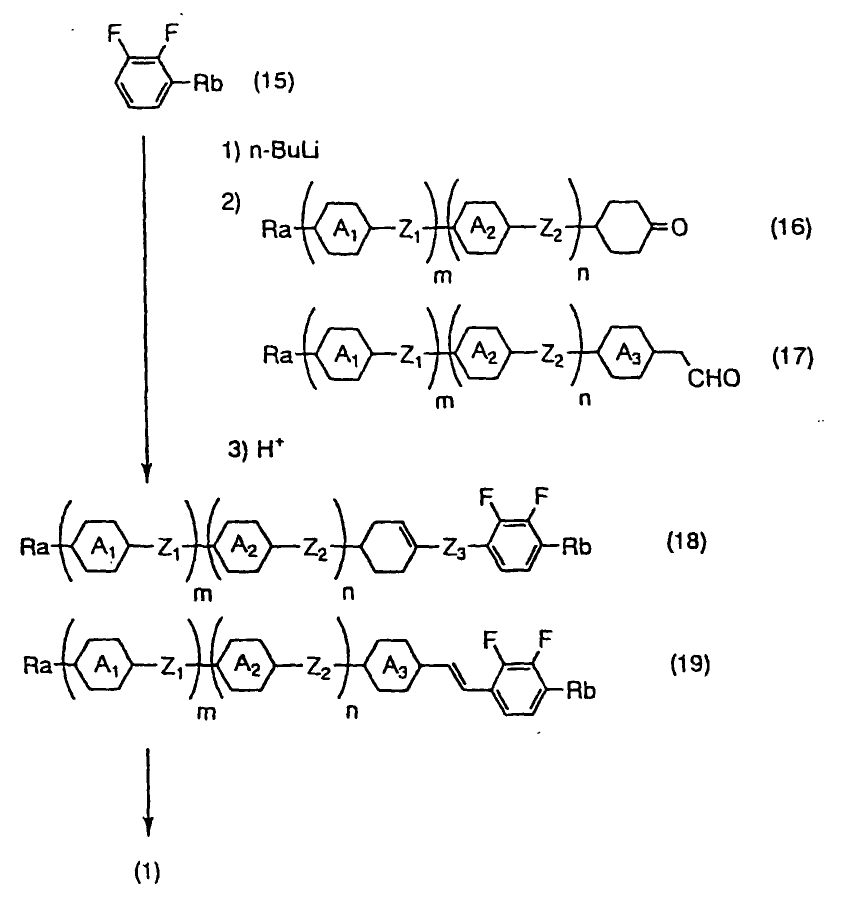

That is, cyclohexene derivative (18) or vinylene derivative (19) can be produced by reacting 4-substituted-2,3-difluorobenzene (15) with n-, sec-, or t-butyl lithium, reacting with cyclohexanone derivative (16) or aldehyde derivative (17), and then subjecting to a dehydration reaction under an acidic condition. Compounds of the general formula (1) can be produced by hydrogenating the double bond in the derivative (18) or (19). The hydrogenation can be performed by a catalytic reducing reaction under hydrogen gas atmosphere in the presence of a catalyst.

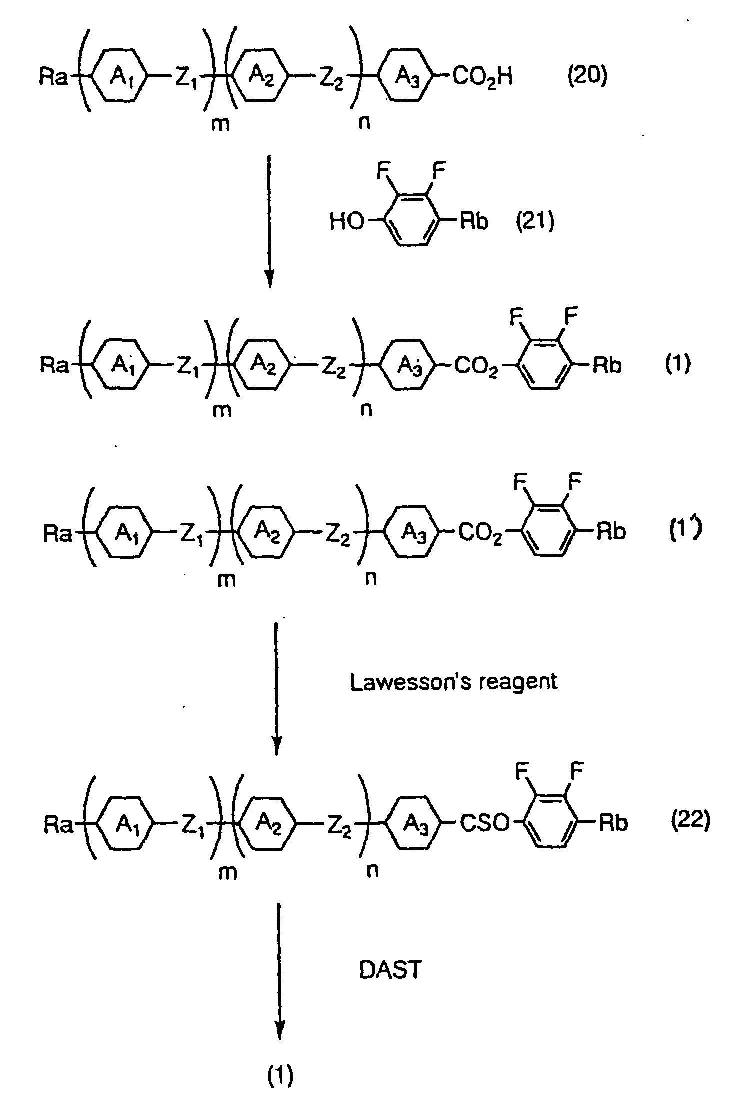

Compounds of the general formula (1) wherein bonding group is ester group can be produced by reacting carboxylic acid derivative (20), which can be prepared by methods known in the literatures, with one of various 4-substituted 2,3-difluorophenols and then subjecting to a dehydrocondensation reaction. While the dehydrocondensation reaction can preferably be performed with a known dehydrating agent (for example, dicyclohexylcarbodiimide), the compounds can also preferably be produced by leading the carboxylic acid derivative (20) to an acid chloride, and then reacting with phenol (21) under a basic condition.

Ester derivative (1') can be led to compound (22) with a known sulfurizing agent such as Lawesson's reagent. Compounds of the general formula (1) wherein bonding group is CF₂O group can be produced by fluorinating the thiocarbonyl group in the compound (22) with one of various fluorinating agents.

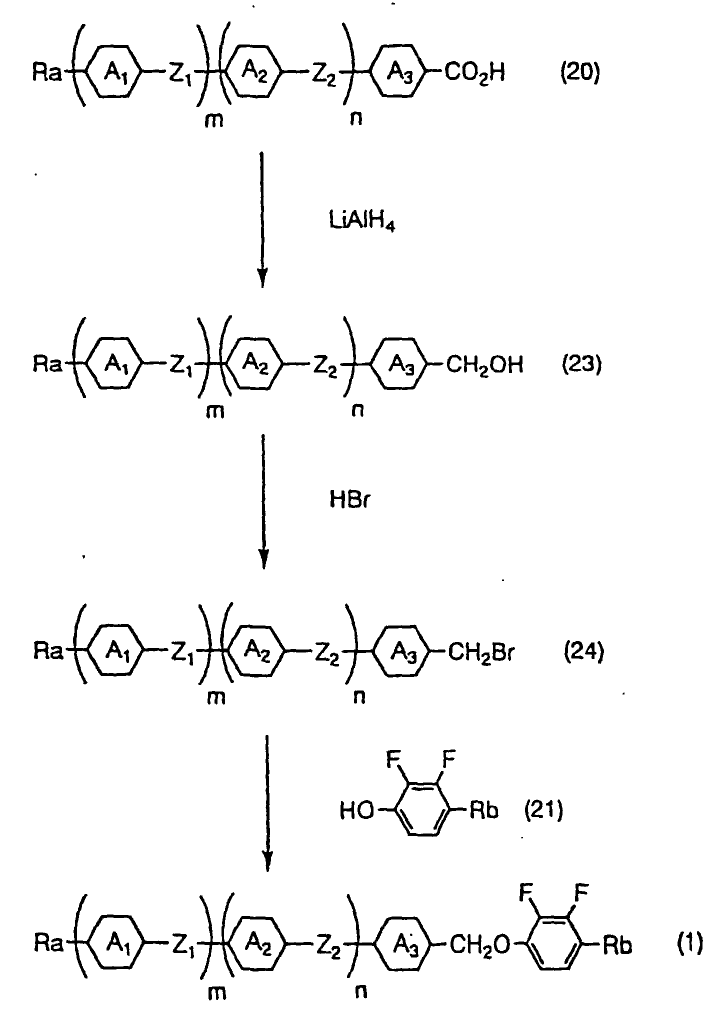

Alcohol derivative (23) can be produced by reducing carboxylic acid (20) with a reducing agent such as lithium aluminum hydride and diisobutyl aluminum hydride. Compounds of the general formula (1) wherein bonding group is CH₂O group can be produced by halogenating the hydroxyl group with one of various halogenating agents and then reacting with phenol (21) to convert into ether.

Introduction of 1,3-dioxane-2,5-diyl or tetrahydropyran at a position of ring A₁ to ring A₄ can readily be performed according to, for example, the method disclosed by H. M. Vorbrodt, R. Eidenschink et al. (H. M. Vorbrodt, J. Prakt. Chem., 323, 902 (1981), R. Eidenschink, DE-OS3306960 (1983)).

Introduction of fluorine atom to a terminal group can be performed according to the known methods described in the literatures. For instance, the purpose can be achieved by fluorinating a corresponding hydroxyl group or carbonyl group with a fluorinating agent represented by diethylaminosulfur trifluoride (J. Org. Chem., 58, 3800 (1993), Tetrahedron Lett., 32, 5963 (1991), and Tetrahedron Asymmetry, 4, 161 (1993)).

Further, introduction of cyclopropane ring to a terminal group can be performed according to the known methods described in the literatures. For instance, the purpose can be achieved by subjecting the double bond portion in a corresponding alkenyl compound to cyclopropanation according to, for example, the method of Simmons-Smith (H. E. Simmons et al., Org. React. 20, 1 (1973)).

Liquid crystal compositions of the present invention are described below. The liquid crystal compositions of the present invention preferably comprise at least one compound expressed by the general formula (1) in the ratio of 0.1 to 99.9 % by weight in order to develop excellent characteristics, and the ratio is more preferably 1 to 60 % by weight.

More specifically, the liquid crystal compositions provided according to the present invention are completed by mixing at least one compound selected from the group consisting of the compounds each expressed by one of the general formulas (2) to (12) depending on the purposes of the liquid crystal compositions to the first component comprising at least one compound expressed by the general formula (1).

As preferable examples of the compounds used in the liquid crystal compositions of the present invention and expressed by one of the general formulas (2) to (4), the compounds of the following formulas can be mentioned. 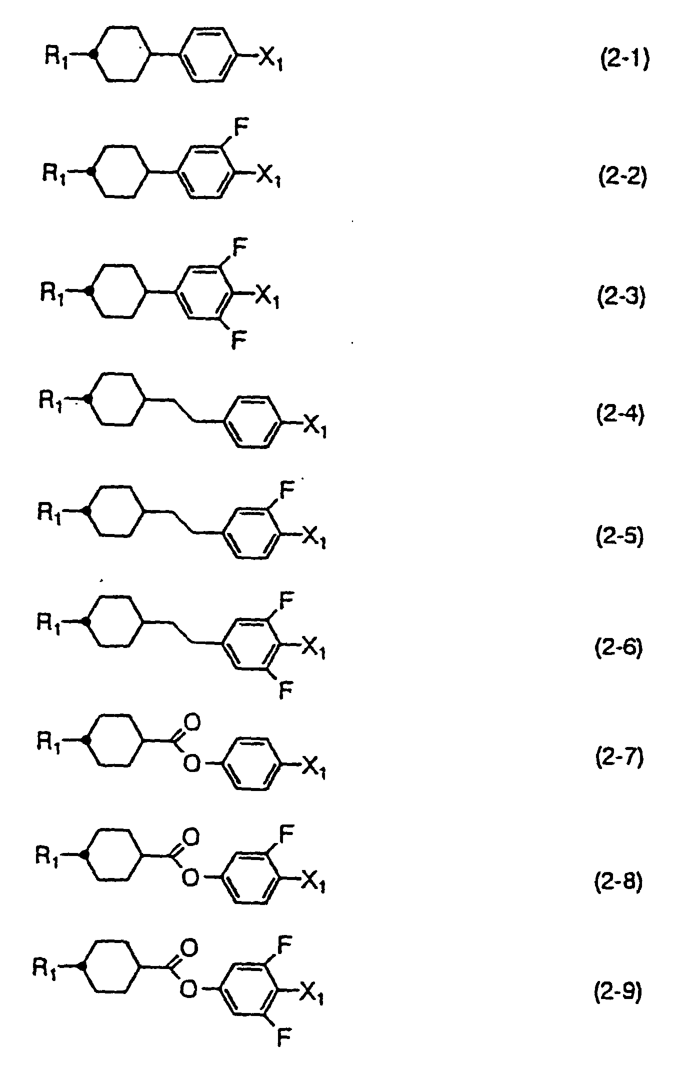 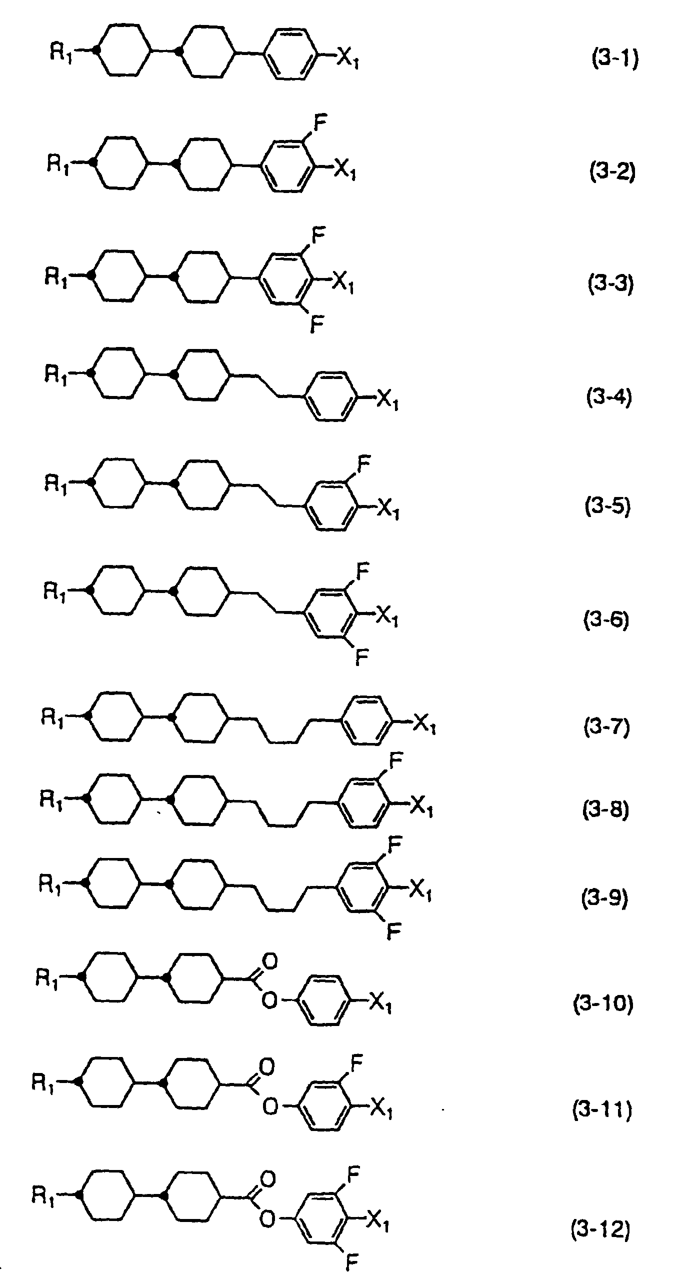 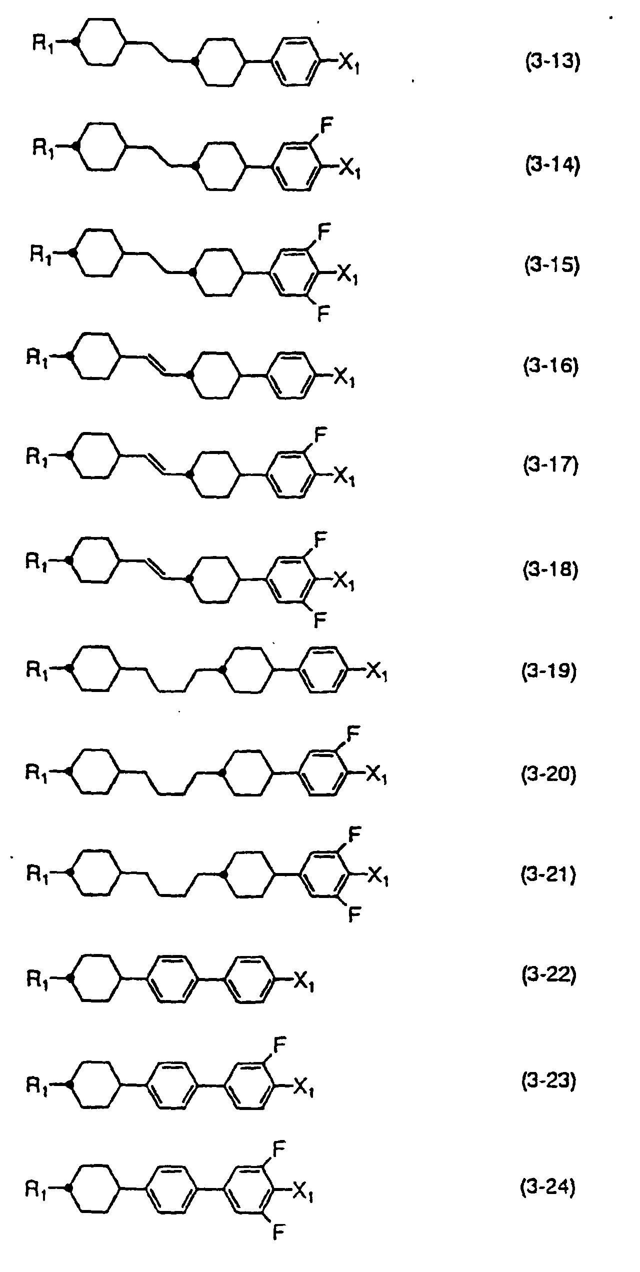 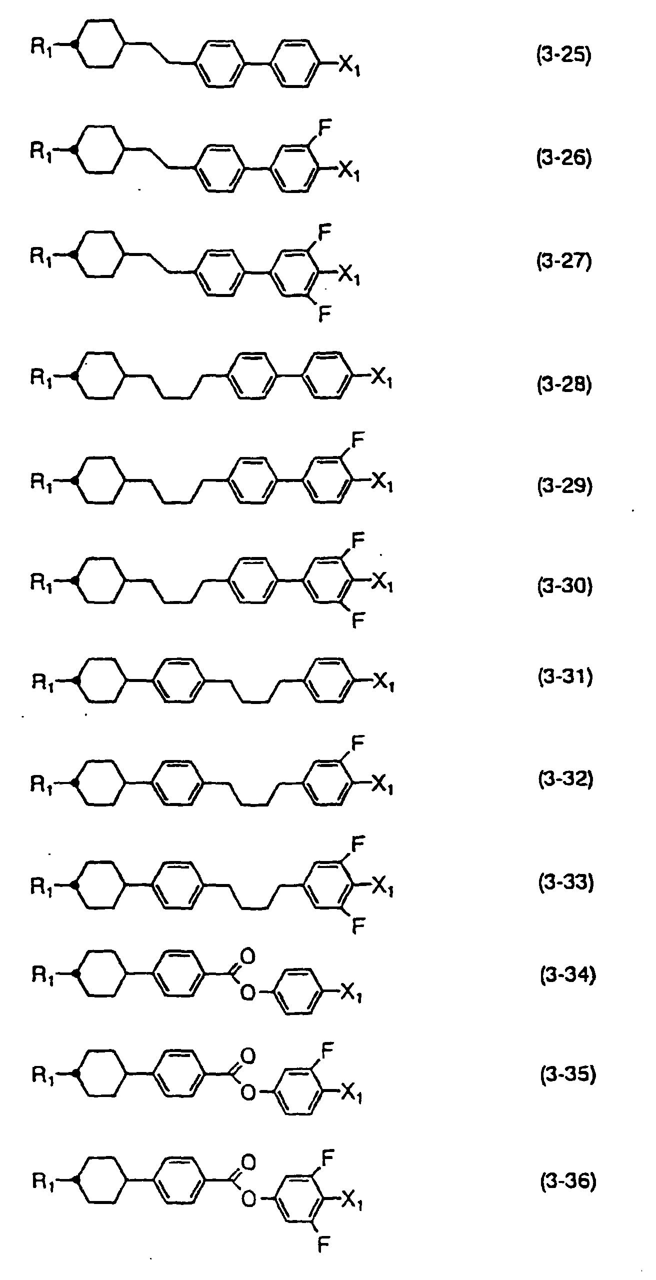 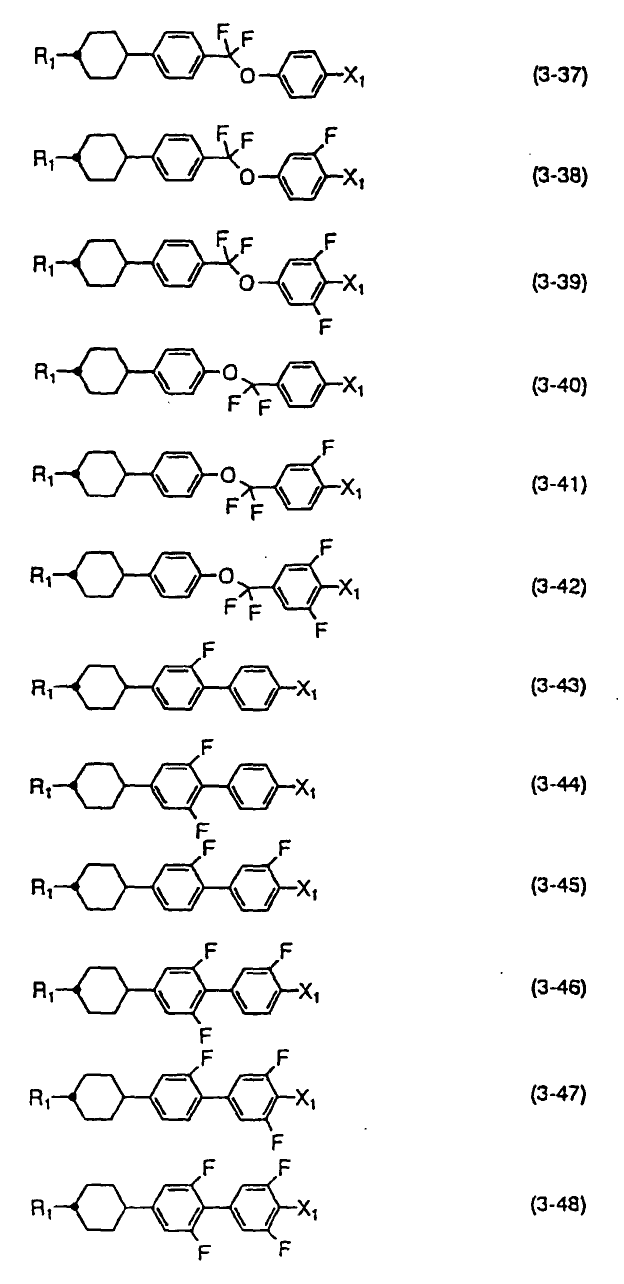 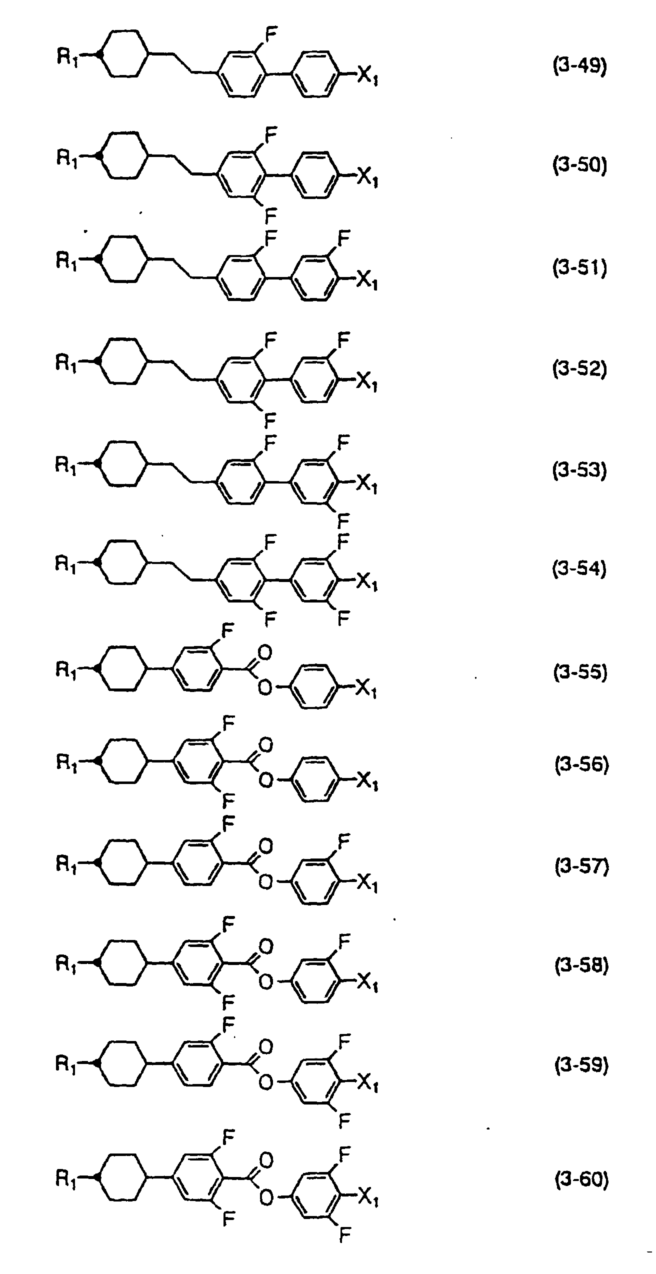 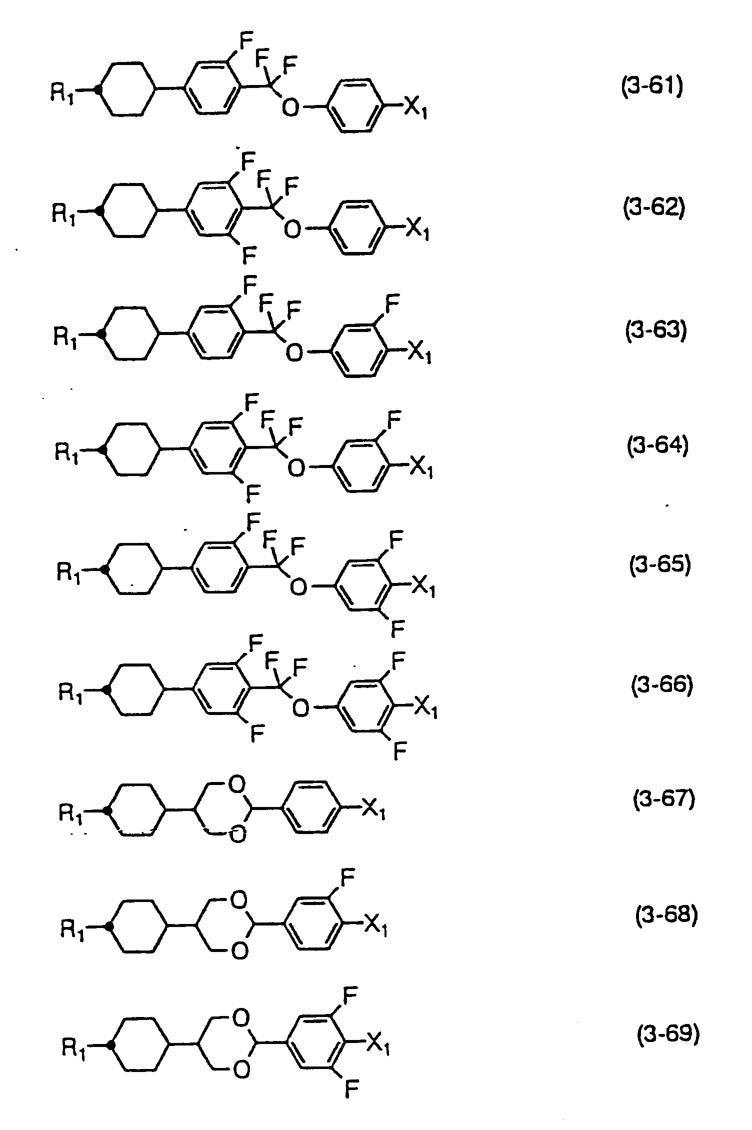 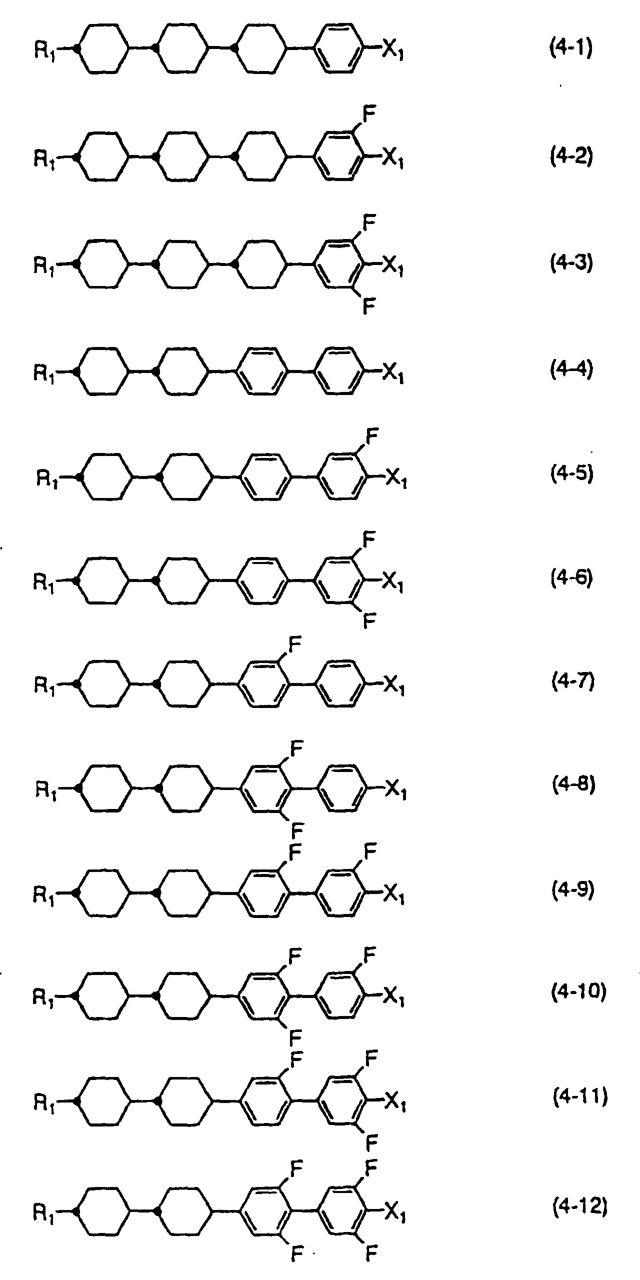 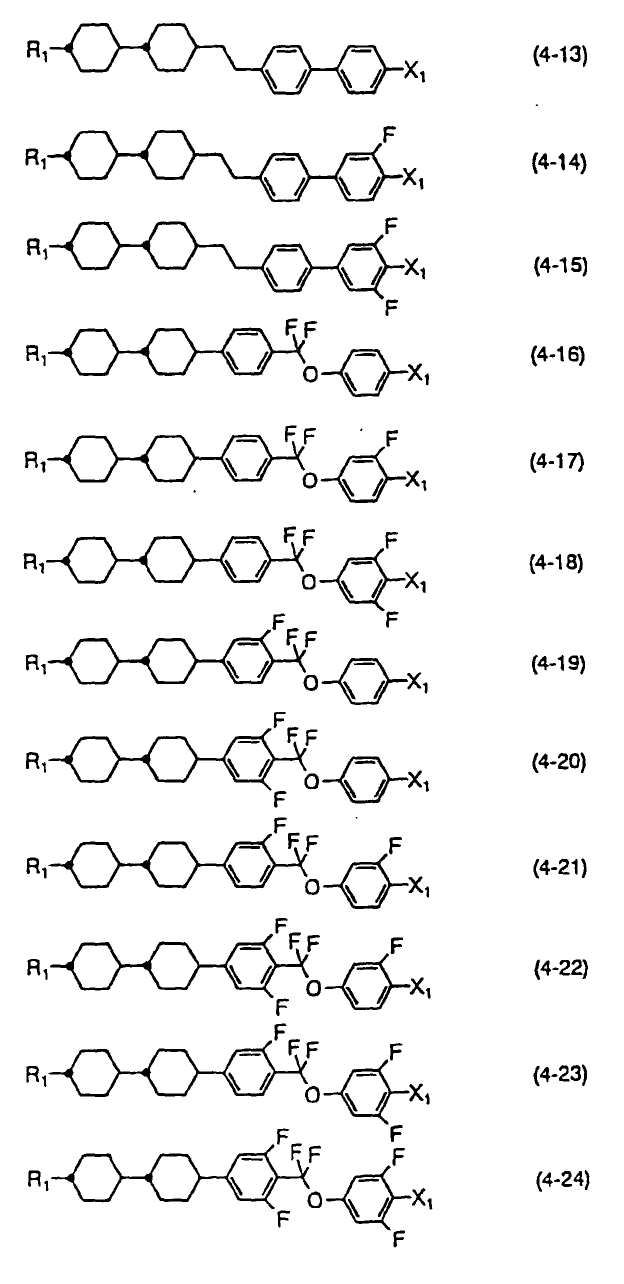 wherein R₁ and X₁ have the same meaning as described above.

Compounds expressed by one of the general formulas (2) to (4) have a positive dielectric anisotropy value and are remarkably excellent in thermal stability and chemical stability. Also, the compounds are extremely useful when liquid crystal compositions for AM (active matrix), particularly for TFT (thin film transistor) are produced of which a high reliability such as a high voltage holding ratio and a large specific resistivity is required in particular.

When liquid crystal compositions for AM are produced, the compounds expressed by one of the general formulas (2) to (4) can be used in the range of 0.1 to 99.9 % by weight based on the total amount of liquid crystal composition, and the range is preferably 10 to 97 % by weight and more desirably 40 to 95 % by weight. Besides, the compounds expressed by one of the general formulas (10) to (12) may further be included for the purpose of adjusting viscosity.

Even when the liquid crystal compositions for STN or TN are produced, the compounds expressed by one of the general formulas (2) to (4) can be used, but the amount to be used is preferably less than 50 % by weight.

As preferable examples of the compounds used in the liquid crystal compositions of the present invention and expressed by the general formula (5) or (6), the compounds of the following formulas can be mentioned. 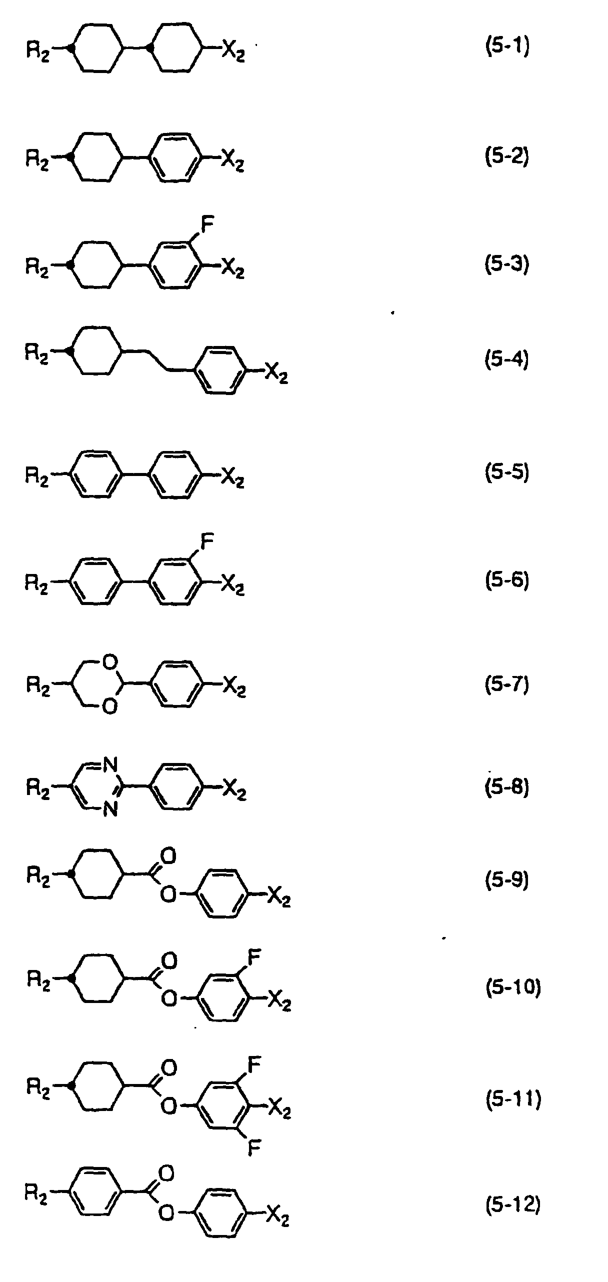 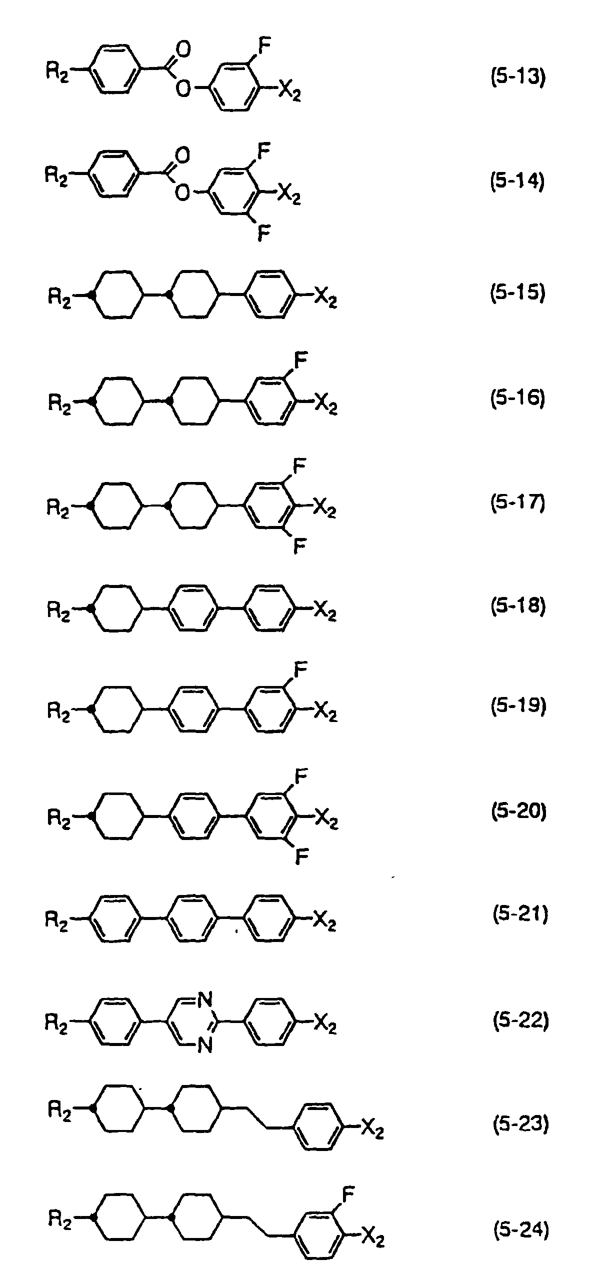 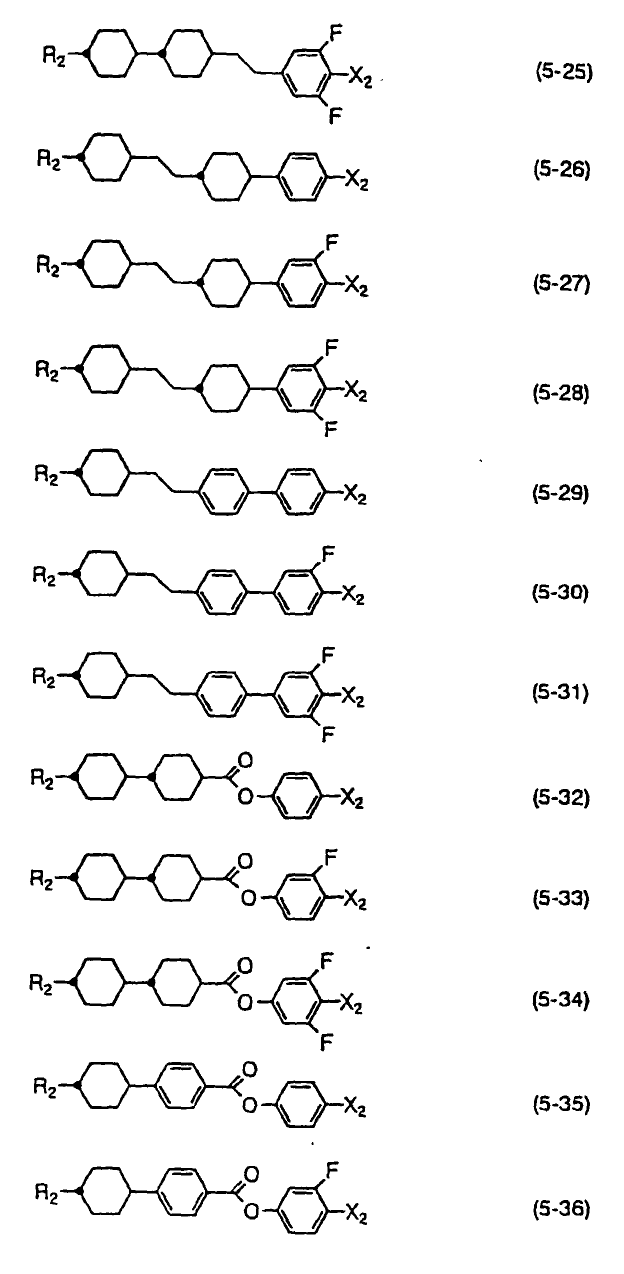 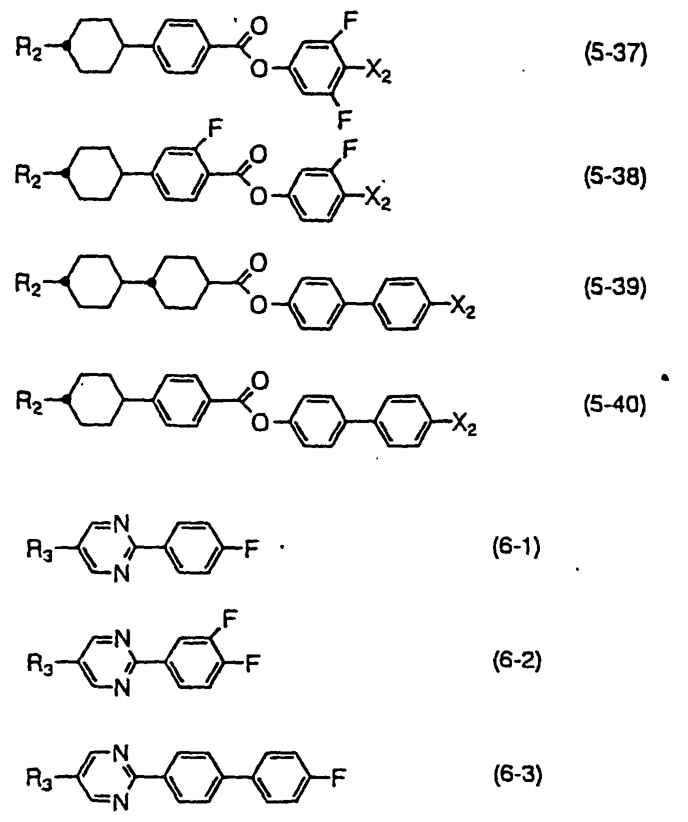 wherein R₂, R₃, and X₂ have the same meaning as described above.

Compounds expressed by the general formula (5) or (6) have a large positive dielectric anisotropy value and are used particularly for the purpose of lowering threshold voltage of liquid crystal compositions. Also, they are used for the purpose of adjusting optical anisotropy value and widening nematic range such as raising clearing point. Further, they are used even for the purpose of improving the steepness of voltage-transmittance curve of liquid crystal compositions for STN or TN.

Compounds expressed by the general formula (5) or (6) are particularly useful when liquid crystal compositions for STN or TN are produced.

When the amount of the compound expressed by the general formula (5) or (6) is increased in liquid crystal compositions, threshold voltage of liquid crystal compositions lowers but viscosity increases. Accordingly, it is advantageous to use the compounds in a large amount since the compositions can be driven at a low voltage, so far as the viscosity of liquid crystal compositions satisfies a required value. When liquid crystal compositions for STN or TN are produced, the compounds expressed by the general formula (5) or (6) can be used in the range of 0.1 to 99.9 % by weight based on the total amount of liquid crystal composition, and the amount is preferably 10 to 97 % by weight and more desirably 40 to 95 % by weight.

As preferable examples of the compounds used in the liquid crystal compositions of the present invention and expressed by one of the general formulas (7) to (9), the compounds of the following formulas can be mentioned. 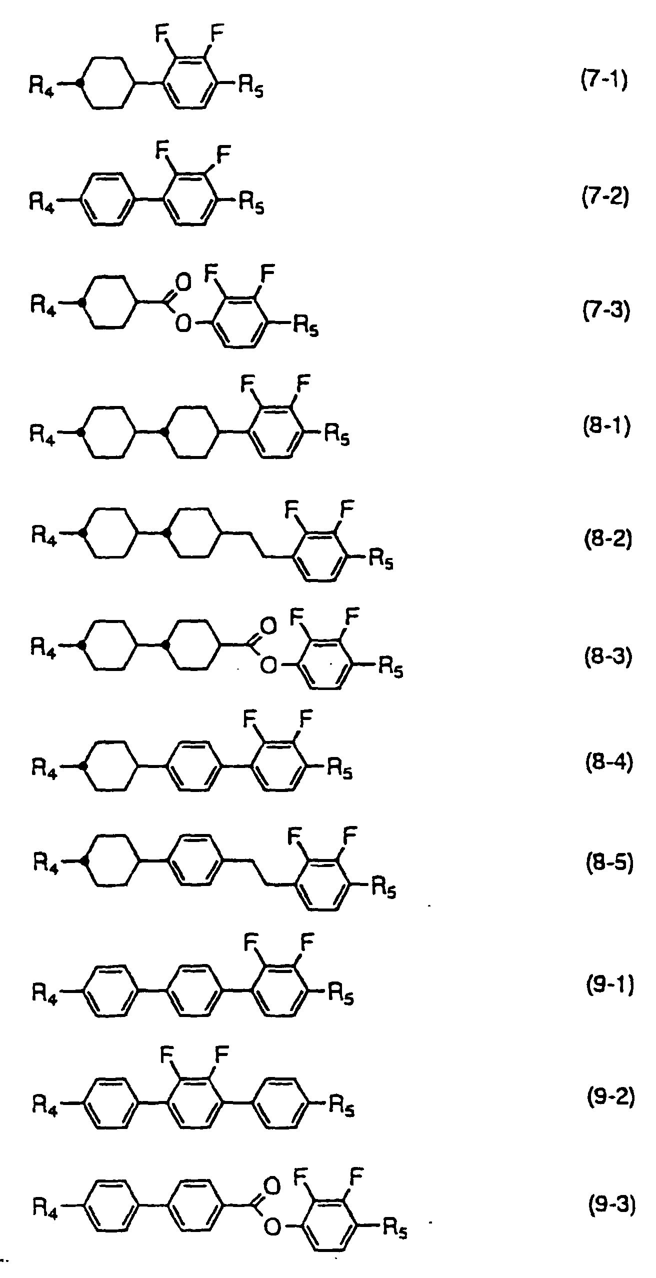 wherein R₄ and R₅ have the same meaning as described above.

Compounds expressed by one of the general formulas (7) to (9) have a negative dielectric anisotropy value. Since the compounds expressed by the general formula (7) are two-ringed compounds, they are used principally for the purpose of adjusting threshold voltage, adjusting viscosity, or adjusting optical anisotropy value. Compounds expressed by the general formula (8) are used for the purpose of widening nematic range such as raising clearing point, or for the purpose of adjusting optical anisotropy value. Compounds expressed by the general formula (9) are used for the purpose of adjusting optical anisotropy value.

Compounds expressed by one of the general formulas (7) to (9) are used principally for the liquid crystal compositions having a negative dielectric anisotropy value. When the amount of the compounds expressed by one of the general formulas (7) to (9) is increased in liquid crystal compositions, threshold voltage of liquid crystal compositions lowers and viscosity increases. Accordingly, it is desirable to use the compounds in a small amount so far as the compositions satisfy a required value of threshold voltage. However, since the absolute value of dielectric anisotropy of the compounds expressed by one of the general formulas (7) to (9) is lower than 5, low voltage driving sometimes becomes impossible when the amount is less than 40 % by weight.

When the liquid crystal compositions used for TFT and having a negative dielectric anisotropy value are produced, the compounds expressed by one of the general formulas (7) to (9) are preferably used in a range of more than 40 % by weight based on the total amount of liquid crystal composition, and the amount is more preferably 50 to 95 % by weight.

Further, the compounds expressed by one of the general formulas (7) to (9) are sometimes mixed to liquid crystal compositions having a positive dielectric anisotropy value for the purpose of improving the steepness of the voltage-transmittance curve by controlling elastic constants. In this case, the compounds expressed by one of the general formulas (7) to (9) are preferably contained in the liquid crystal compositions in an amount of less than 30 % by weight.

As preferable examples of the compounds used in the liquid crystal compositions of the present invention and expressed by one of the general formulas (10) to (12), the compounds of the following formulas can be mentioned. 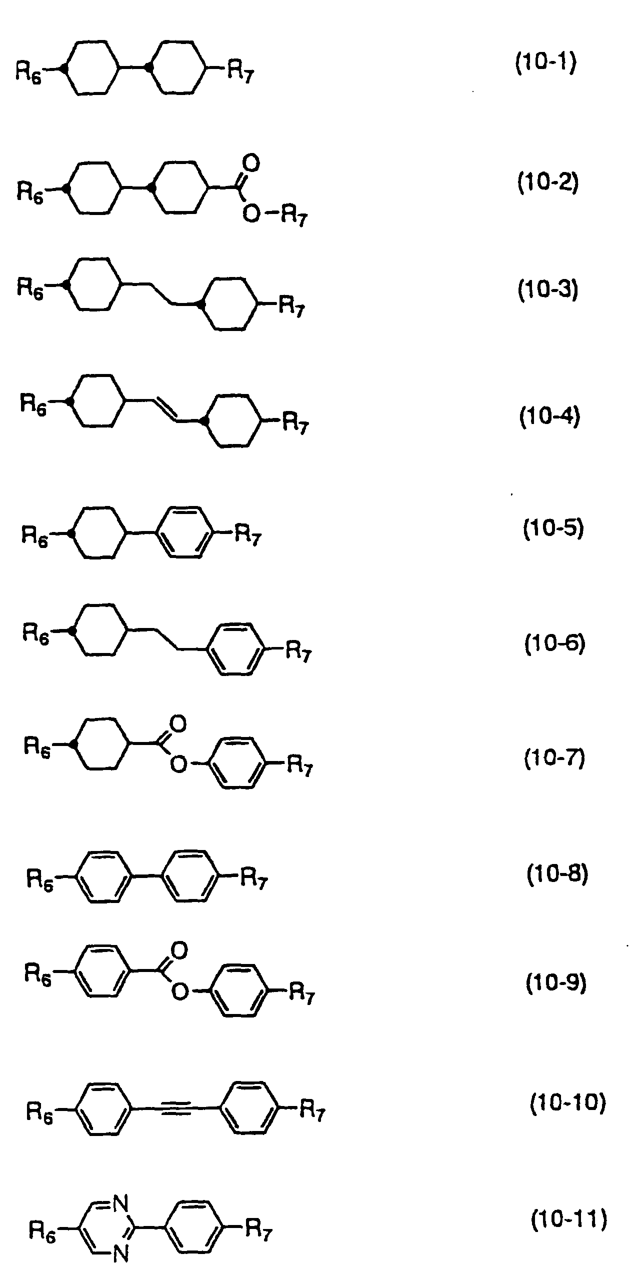 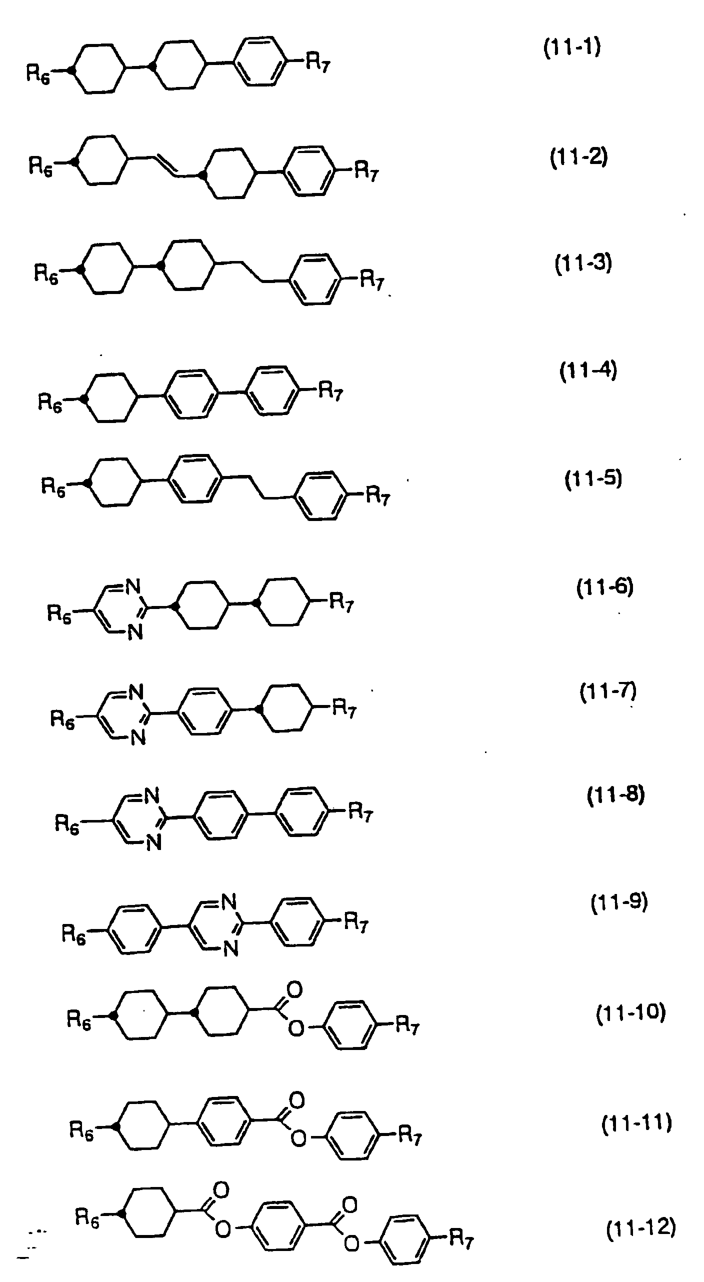 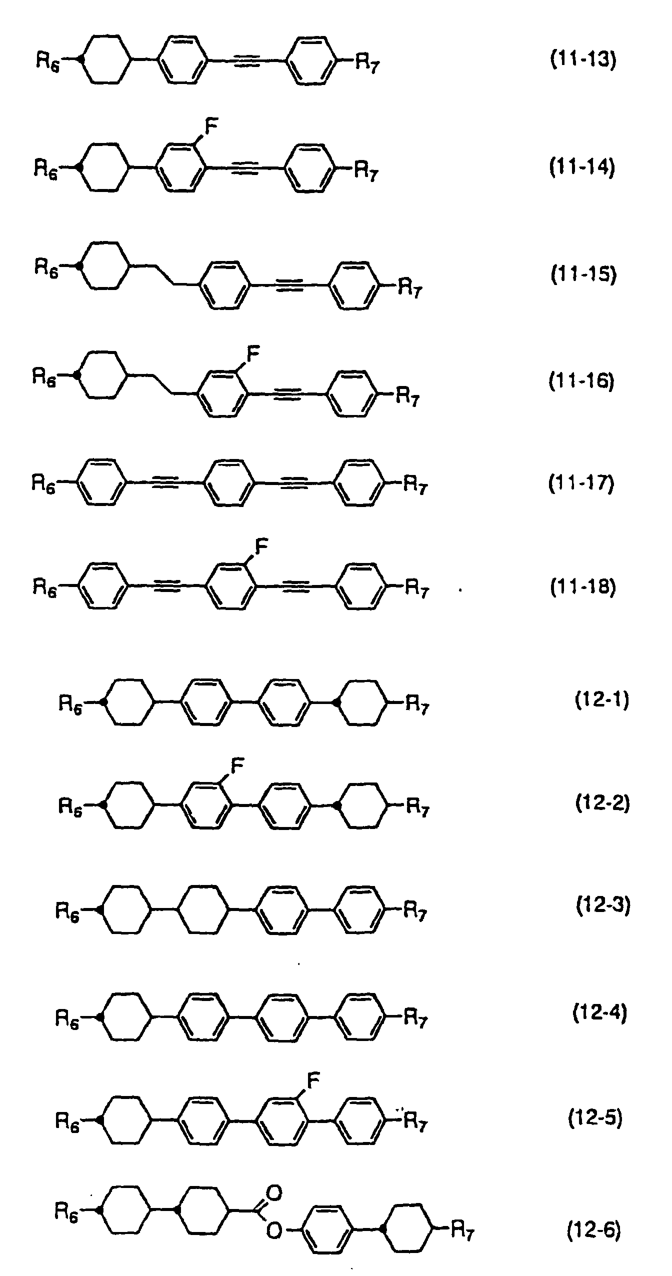 wherein R₆ and R₇ have the same meaning as described above.

Compounds expressed by one of the general formulas (10) to (12) have a small absolute value, close to zero, of dielectric anisotropy. Compounds expressed by the general formula (10) are used principally for the purpose of adjusting viscosity or adjusting optical anisotropy value. Further, compounds expressed by the general formula (11) or (12) can be used for the purpose of widening nematic range such as raising clearing point, or adjusting optical anisotropy value.

When the amount of the compounds expressed by one of the general formulas (10) to (12) is increased in liquid crystal compositions, threshold voltage of the liquid crystal compositions increases but viscosity reduces. Accordingly, it is desirable to use the compound in a large amount in the range wherein threshold voltage of liquid crystal compositions satisfies a required value. When liquid crystal compositions for TFT are produced, the compounds expressed by one of the general formulas (10) to (12) are preferably contained in liquid crystal compositions in an amount of less than 40 % by weight, and the amount is more preferably less than 35 % by weight. Further, when liquid crystal compositions for STN or TN are produced, the compounds expressed by one of the general formulas (10) to (12) are contained in liquid crystal compositions in an amount less than 70 % by weight, and more desirably less than 60 % by weight.

In liquid crystal compositions for STN, TFT, or others, an optically active compound is usually added for the purpose of inducing helical structure of liquid crystals to adjust required twist angle and to prevent reverse twist. Even in the liquid crystal compositions of the present invention, any known optically active compound can be used for such purposes. As examples of preferable optically active compounds, the compound of the following formulas can be mentioned:

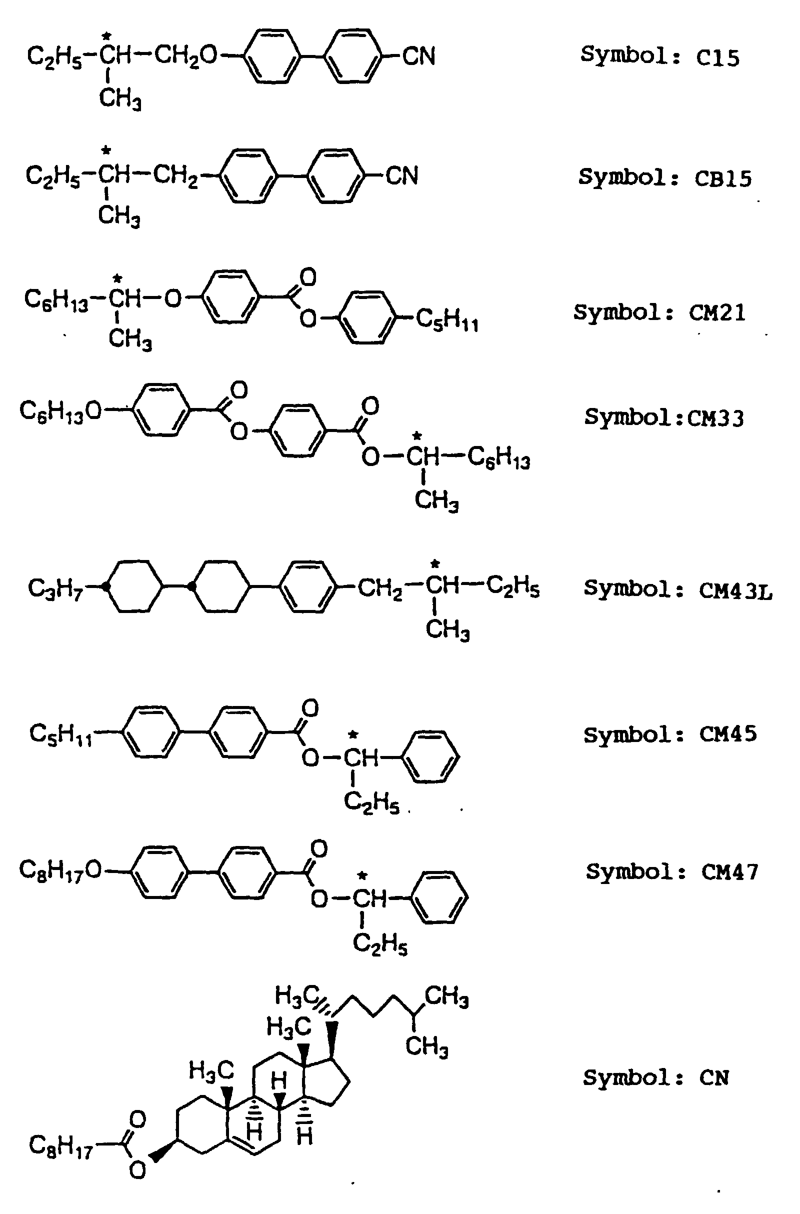

Usually, these optically active compounds are added to the liquid crystal compositions of the present invention to adjust the pitch of the twist. Pitch of the twist is preferably adjusted in the range of 40 to 200 µm in the case of liquid crystal compositions for TFT or TN, and preferably adjusted in the range of 6 to 20 µm in the case of liquid crystal compositions for STN. In the case of liquid crystal compositions for bistable TN, it is preferably adjusted in the range of 1.5 to 4 µm. Besides, two or more kind of optically active compounds may be added for the purpose of adjusting the dependency of the pitch on temperature.

Liquid crystal compositions of the present invention can be used as ones for GH (guest-host) mode by adding a dichroic dye such as merocyanine type, styryl type, azo type, azomethine type, azoxy type, quinophthalone type, anthraquinone type, and tetrazine type thereto. Further, the liquid crystal compositions can be used as NCAP which is prepared by the microencapsulation of a nematic liquid crystal, or as liquid crystal compositions for polymer dispersed liquid crystal display devices (PDLCD) represented by polymer net work liquid crystal display devices (PNLCD) prepared by forming a polymer of three-dimensional reticulated structure in a liquid crystal. Still further, the liquid crystal compositions of the present invention can be used as ones for ECB (electrically controlled birefringence) mode or dynamic scattering (DS) mode.

Liquid crystal compositions of the present invention can be produced by conventional methods. Generally, a method is adopted in which various components are dissolved in one another at a high temperature.

### BEST MODE FOR CARRYING OUT THE INVENTION

The present invention will be described in more detail below with reference to Examples.

### Example 1

Preparation of 1-(4-propylcyclohexyl)-4-(2,3-difluoro-4-(3-fluoropropyloxy)phenyl)cyclohexane (Compound expressed by the general formula (1) wherein Ra is propyl group, Rb is 3-fluoropropyloxy group, each of A₂ and A₃ is cyclohexane-1,4-diyl, A₄ is 2,3-difluorophenyl, Z₂ and Z₃ are single bonds, m is 0, and n is 1)

First, 2,3-difluoanisole (0.5 mol) was dissolved in THF (300 ml) and cooled down to -65°C. sec-Butyl lithium (1.06M THF solution) in an amount corresponding to 0.55 mol was added dropwise thereto slowly in the range which did not exceed that temperature. After it was stirred for 1 hour, a solution of 4-(4-propylcyclohexyl)cyclohexanone (0.55 mol) in THF (100 ml) was added dropwise thereto, gradually warmed up to room temperature (about 18°C), and stirred for 3 hours.

Saturated aqueous ammonium chloride solution (100 ml) was added to the reaction solution and stirred for 10 minutes. It was extracted twice with ethyl acetate (300 ml), and the organic layer was washed with saturated aqueous sodium chloride solution (50 ml) and dried over anhydrous magnesium sulfate. The solid was filtered off and concentrated under a reduced pressure. To the red tar-like residue thus obtained were added toluene (200 ml) and p-toluenesulfonic acid (0.3 mol), and heated to reflux for 3 hours while removing the generated water to the outside of the reaction system.

The reaction solution was cooled with ice, washed twice with saturated aqueous sodium chloride solution (100 ml), and dried over anhydrous magnesium sulfate. The solid was filtered off, and the filtrate was concentrated under a reduced pressure to obtain a red tar-like residue. Ethanol (300 ml), ethyl acetate (200 ml), and Raney nickel (15 g) were added thereto and stirred under hydrogen gas atmosphere (1 atom) for 7 hours. After confirmed that the reaction solution does not absorb hydrogen gas, the catalyst was filtered off and the filtrate was concentrated.

The residue thus obtained was purified by silica gel column chromatography (eluent: toluene) and then by recrystallization (thrice with 500 ml of ethanol) to obtain 1-(4-propyl-cyclohexyl)-4-(2,3-difluoro-4-methoxyphenyl)cyclohexane (0.12 mol). This compound exhibited a liquid crystal phase, and its phase transition temperatures were as follows:
Cr 89.1 N 178.0 I

Solution of 1-(4-propylcyclohexyl)-4-(2,3-difluoro-4-methoxyphenyl)cyclohexane (0.10 mol) in dichloromethane (200 ml) was cooled down to -71°C, and boron tribromide (0.12 mol) was slowly added dropwise thereto, and stirred as it was for 5 hours. The reaction product was slowly put in water (300 ml), extracted thrice with diethyl ether (200 ml), and dried over anhydrous magnesium sulfate. The solid product was filtered off , and the filtrate was concentrated to obtain a white residue.

The residue thus obtained was purified by recrystallization (toluene 100 ml) to obtain 2,3-difluoro-4-(4-(4-propylcyclohexyl)cyclohexyl)phenol (0.041 mol).

A mixture prepared by adding 3-fluoropropylbromide (0.05 mol), potassium carbonate (0.06 mol), and ethanol (100 ml) to the 2,3-difluoro-4-(4-(4-propylcyclohexyl)cyclohexyl)phenol obtained in the procedures described above (0.04 mol) was heated to reflux for 6 hours. Toluene was added to the residue which was obtained by removing most of the solvent under a reduced pressure, and sufficiently stirred. The toluene layer was washed with water (500 ml) and dried over anhydrous magnesium sulfate. The solid was filtered off and the filtrate was concentrated under a reduced pressure to obtain a white solid residue.

This product was purified by silica gel column chromatography (eluent: heptane) and then by recrystallization (twice with 30 ml of heptane) to obtain the subject compound (0.023 mol).
¹H-NMR (CDCl₃) δ: 6.95-6.57 (m, 2H), 4.65 (td, 2H), (t, 2H), 2.72 (bt, 1H), 2.45-0.79 (m, 28H)

This product exhibited liquid crystallinity and its phase transition temperatures were as follows:
Melting point (Cr) = 109.3°C
Nematic phase-isotropic liquid phase (T_{NI}) = 168.3°C

### Example 2 (Use Example 1)

Liquid crystal composition (A) comprising the following liquid crystalline compounds in the amount shown below was prepared:

| | |
|---|---|
| 4-ethoxyphenyl=4-propylcyclohexane carboxylate | 17.2 % |
| 4-butoxyphenyl=4-propylcyclohexane carboxylate | 27.6 % |
| 4-ethoxyphenyl=4-butylcyclohexane carboxylate | 20.7 % |
| 4-methoxyphenyl=4-pentylcyclohexane carboxylate | 20.7 % |
| 4-ethoxyphenyl=4-pentylcyclohexane carboxylate | 13.8 % |

Physical properties of this liquid crystal composition were as follows:
T_{NI} = 74.0°C
Δε = -1.3
Δn = 0.087

In the above, T_{NI} indicates the phase transition temperature of nematic phase-isotropic liquid.

Physical properties of liquid crystal composition (B) comprising 85 % by weight of the liquid crystal composition (A) and 15 % by weight of the 1-(4-propylcyclohexyl)-4-(2,3-difluoro-4-(3-fluoropropyl)phenyl)cyclohexane obtained in Example 1 were as shown below. Values in the parentheses show the ones of 1-(4-propylcyclohexyl)-4-(2,3-difluoro-4-(3-fluoropropyl)phenyl)cyclohexane calculated by extrapolation from the mixing ratio.
T_{NI} = 83.0°C
Δε = -1.95 (-4.5)
Δn = 0.097 (0.092)

### Example 3

Following compounds (Compound No. 1 through Compound No. 308) were prepared according to the preparation method described in Example 1 and those described above. Transition temperatures described together indicate the phase transition temperatures of particular compounds themselves, and Δε and Δn show extrapolated values calculated from the physical properties of the liquid crystal compositions obtained by mixing 15 % by weight of the compounds with the composition (A) described above, respectively.

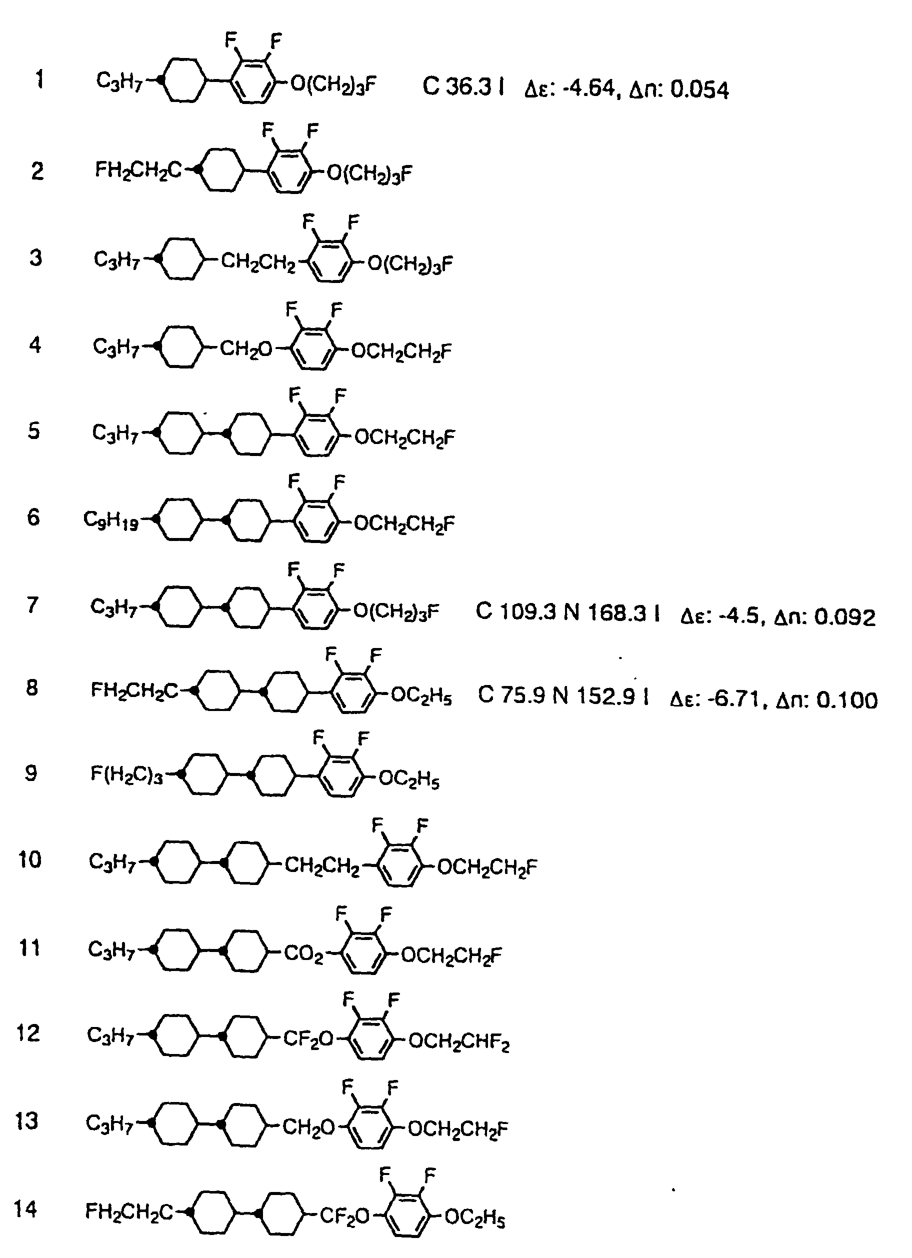

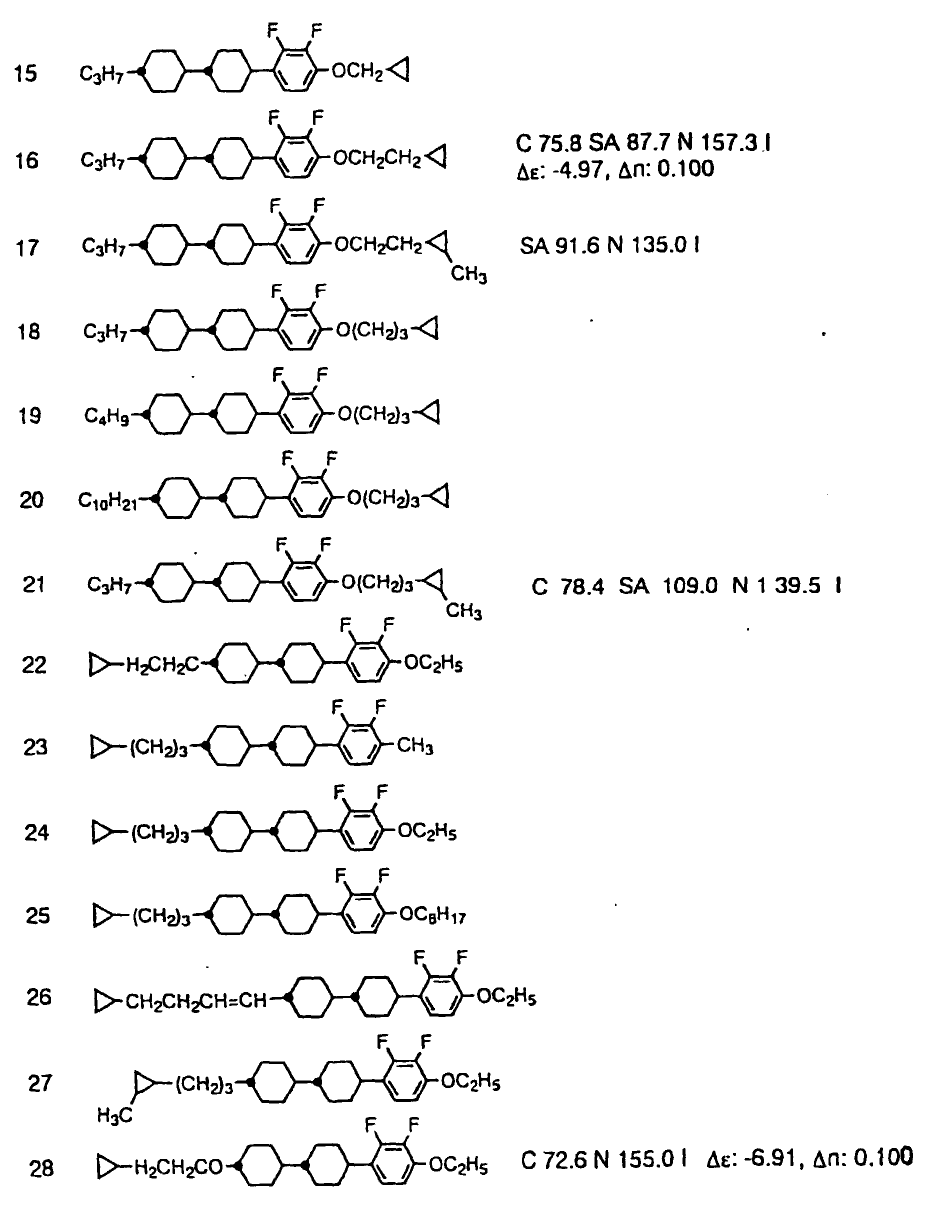

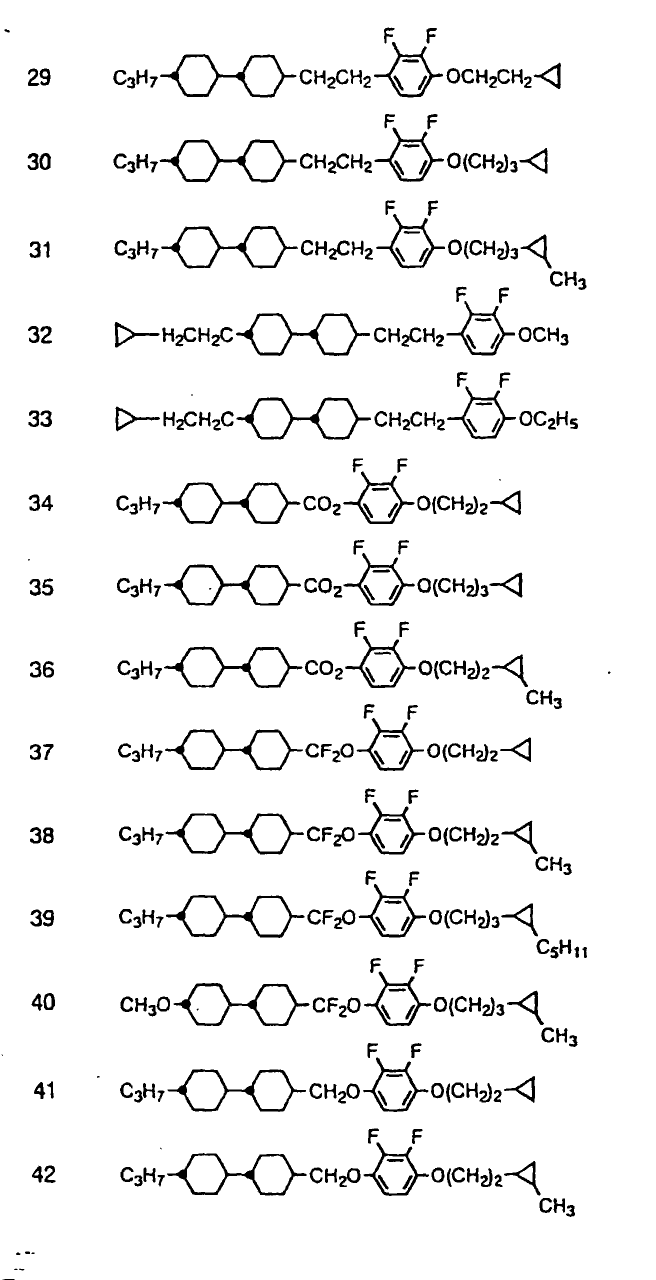

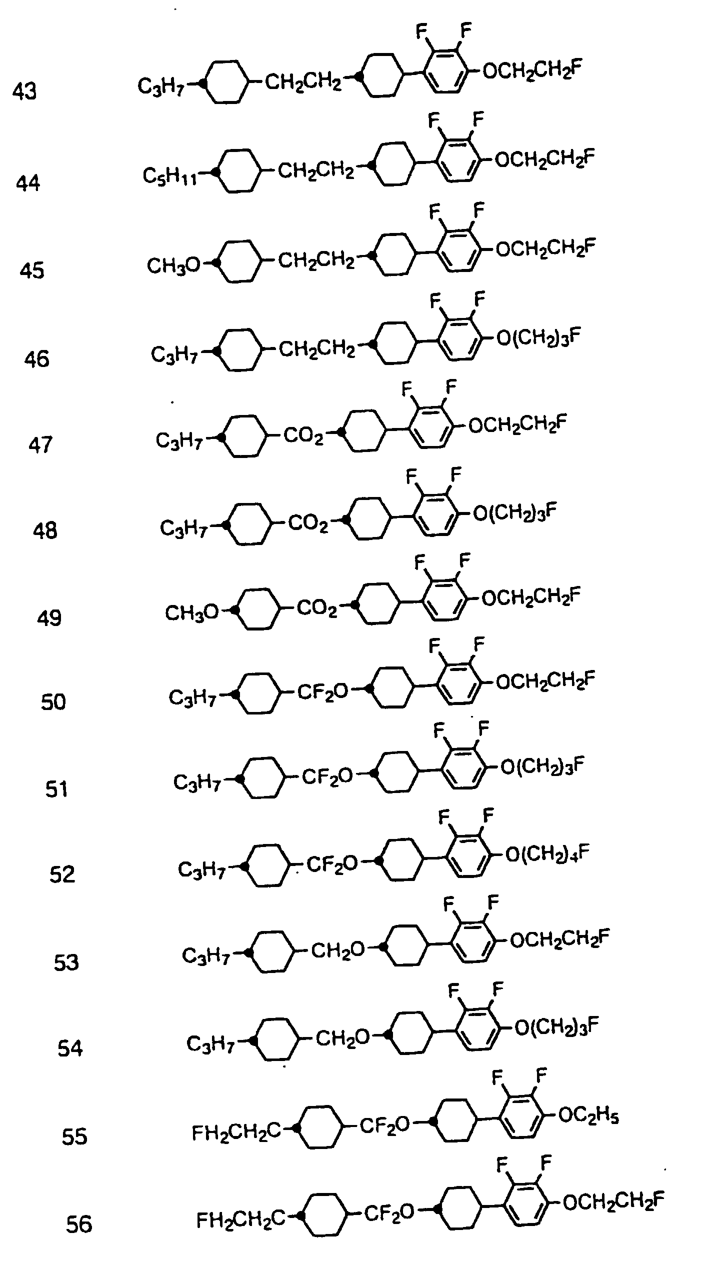

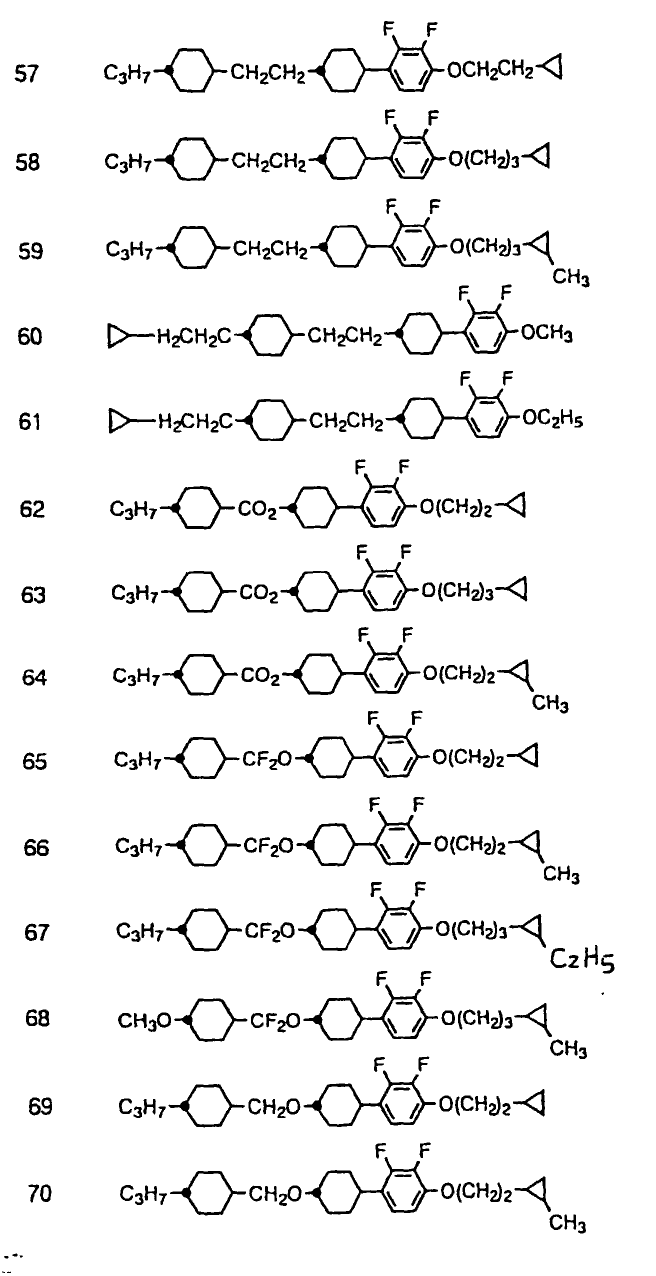

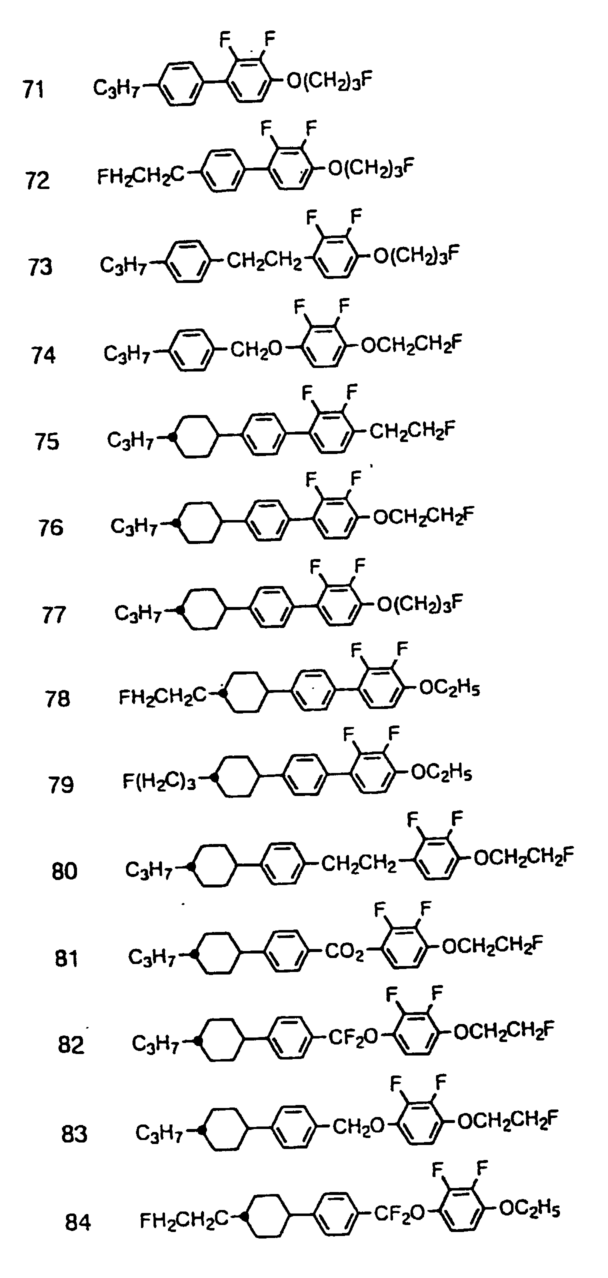

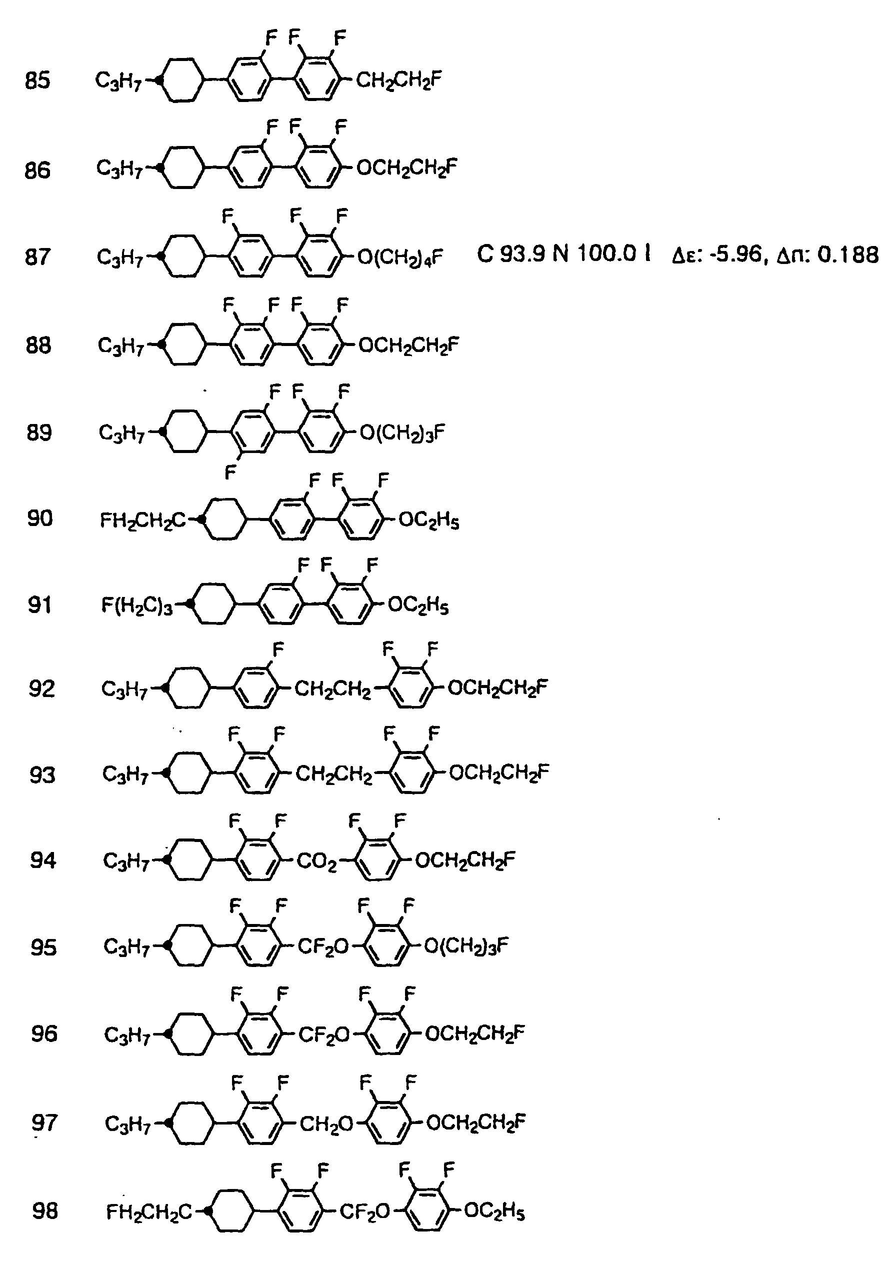

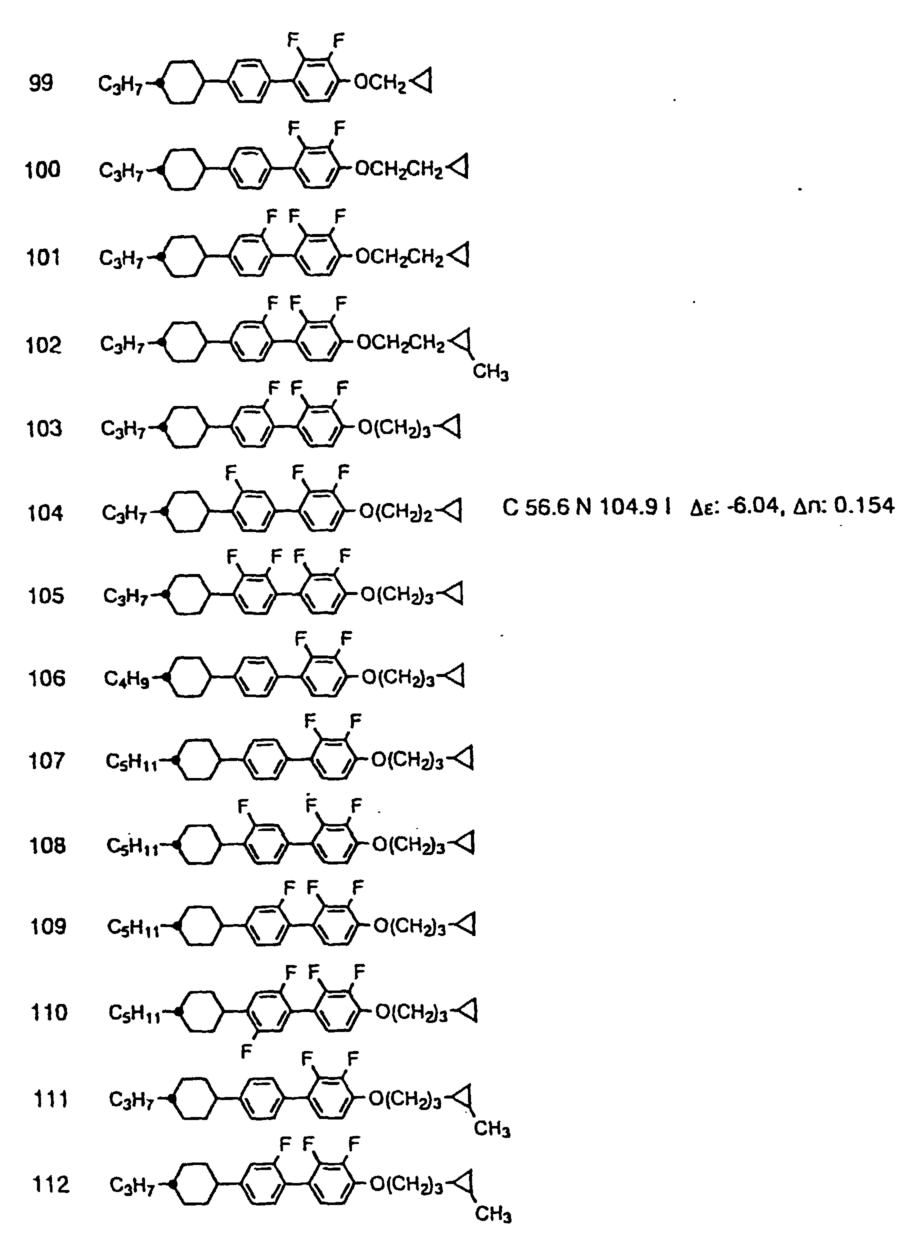

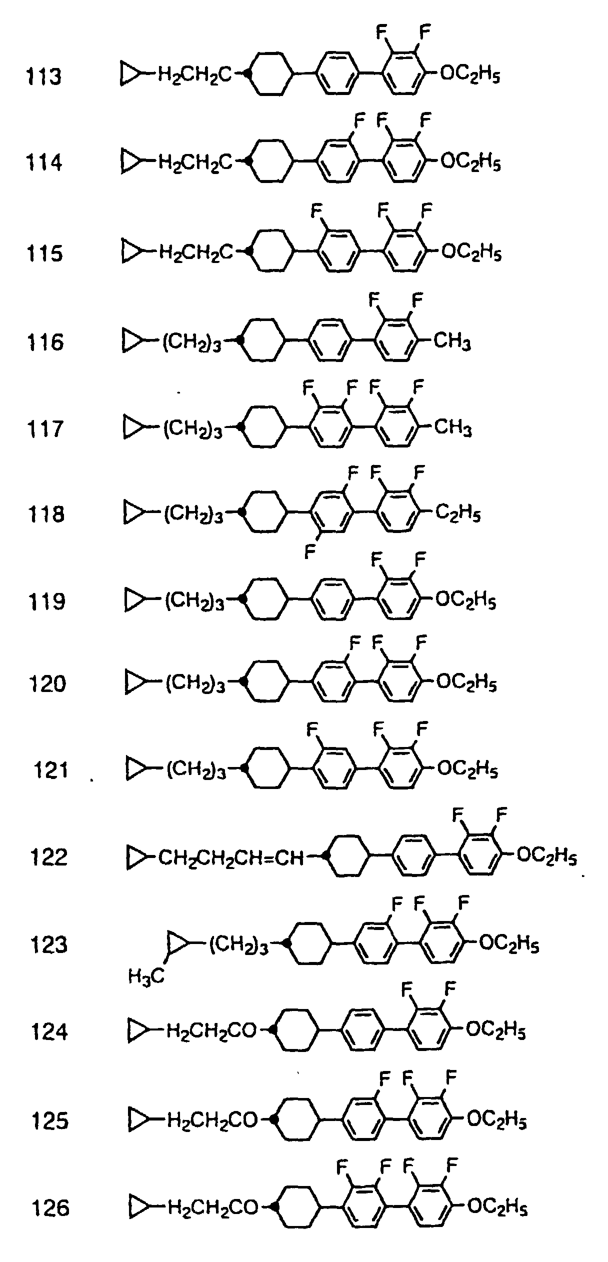

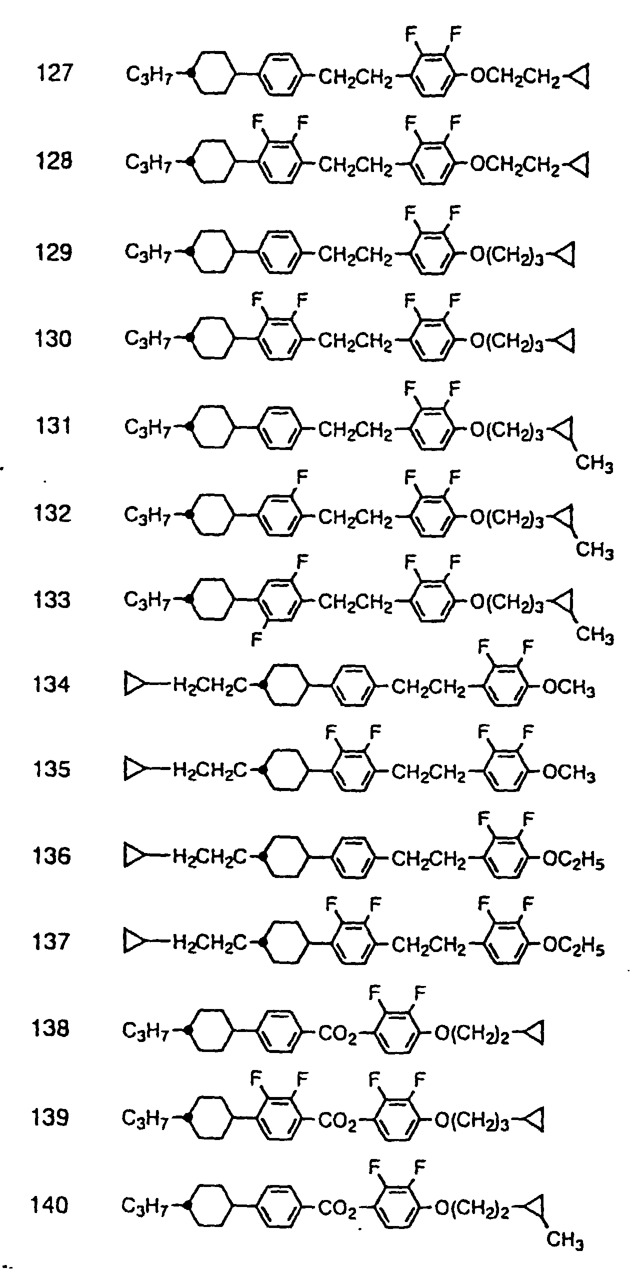

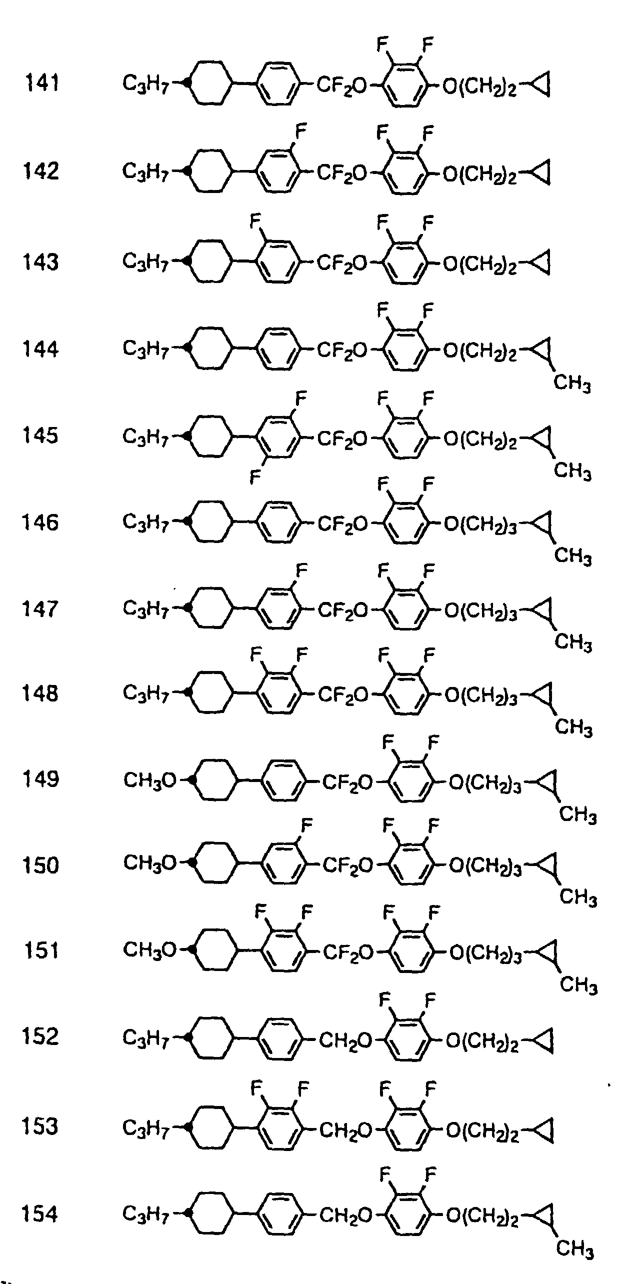

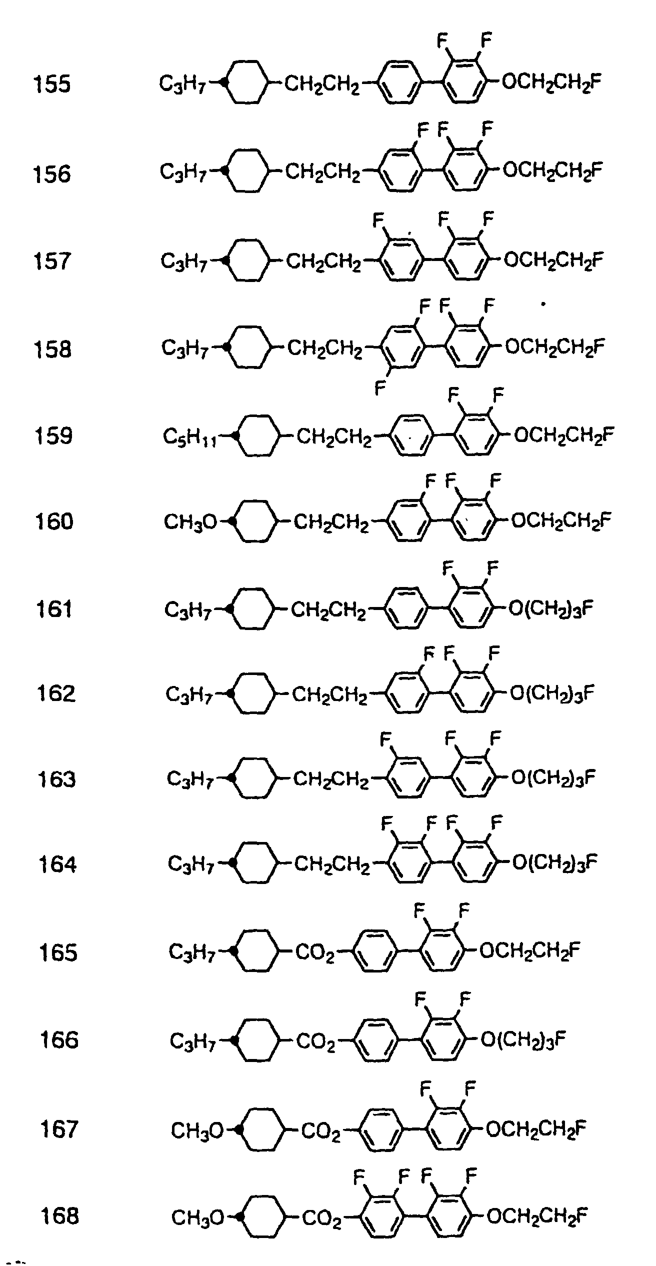

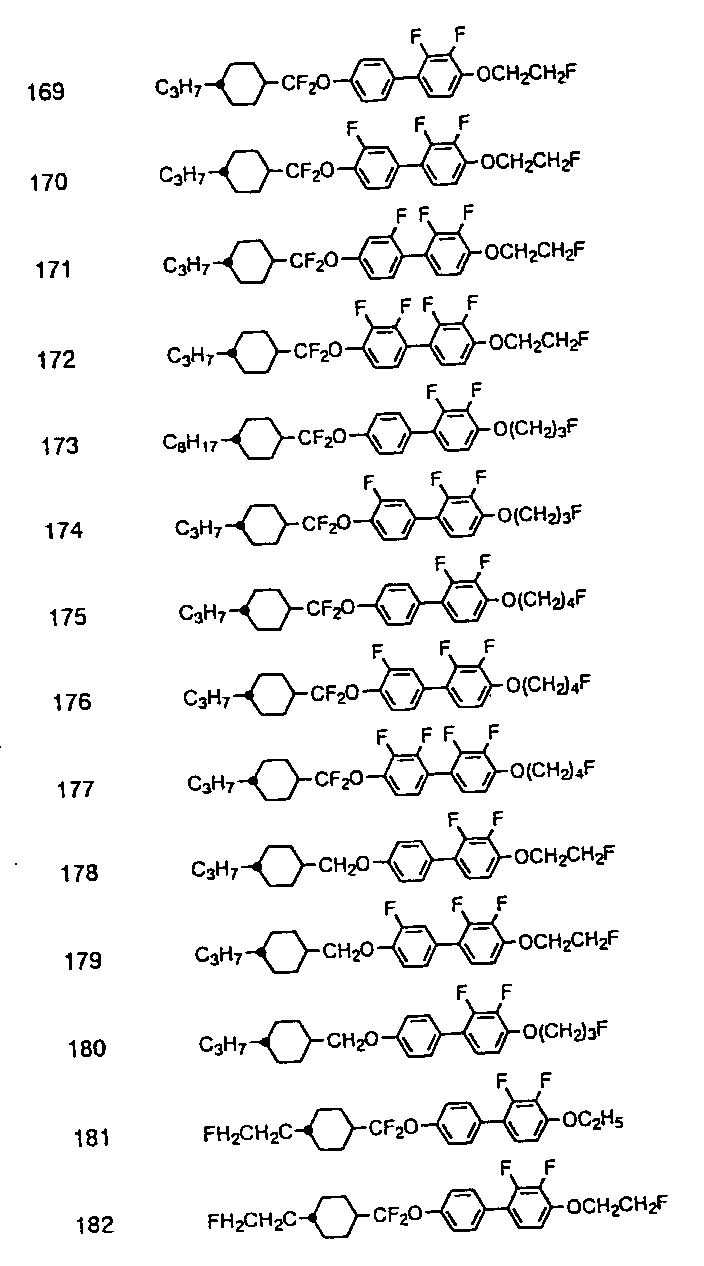

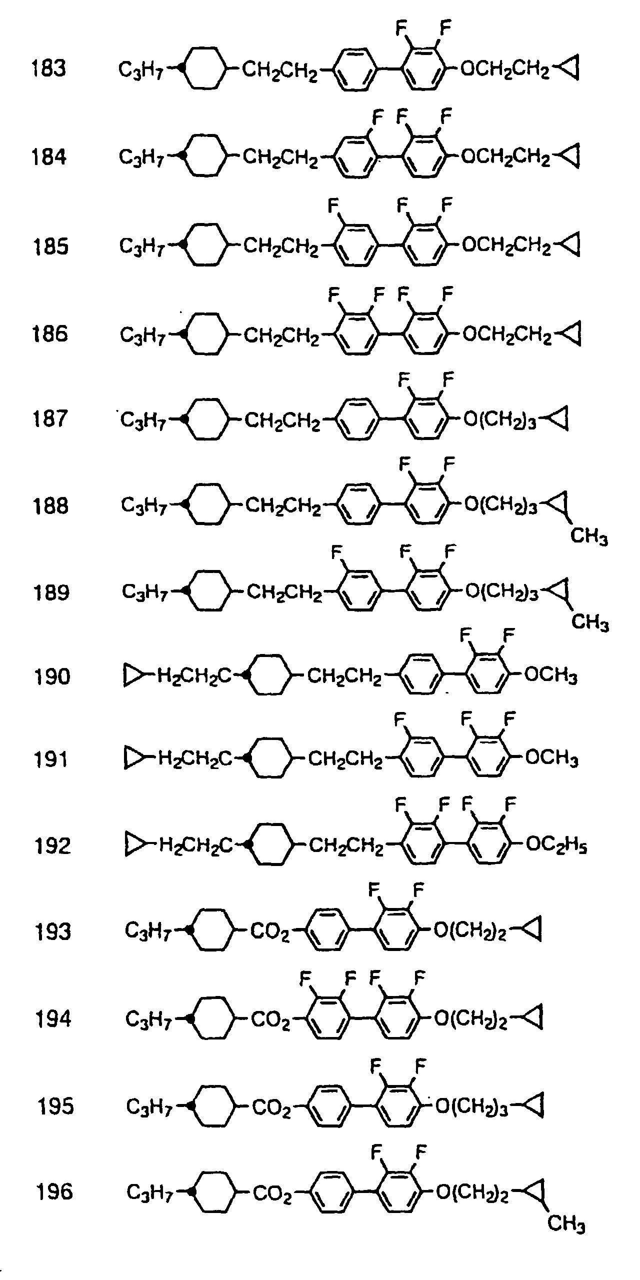

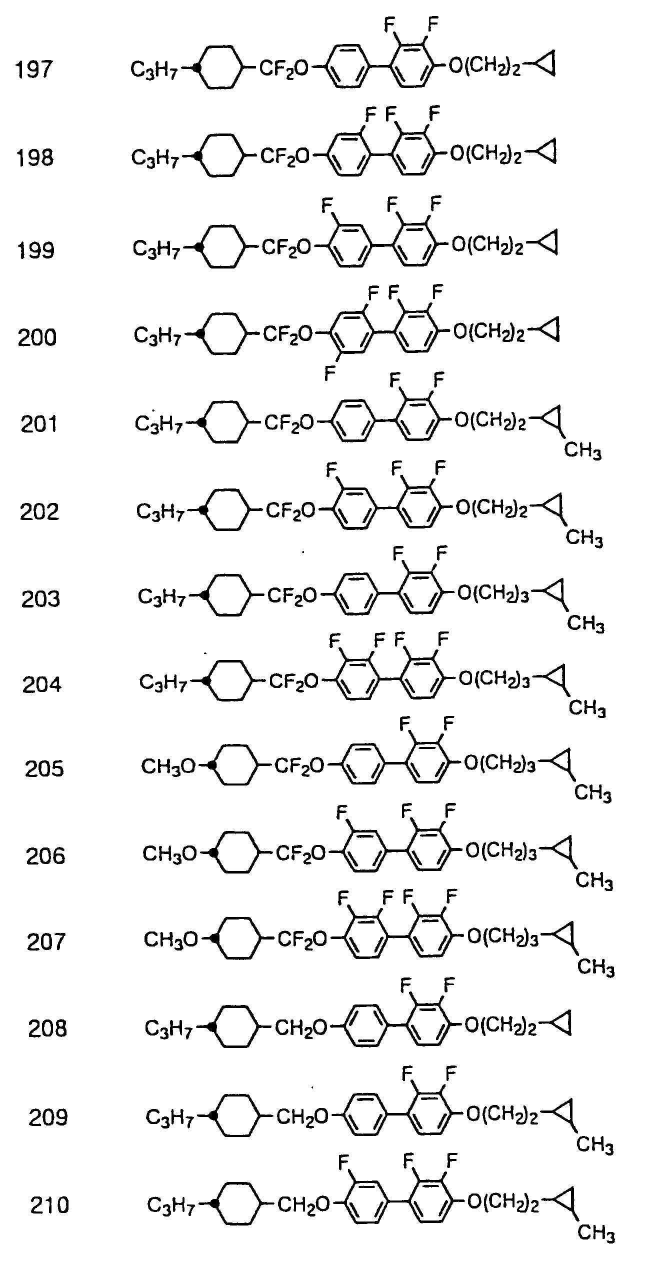

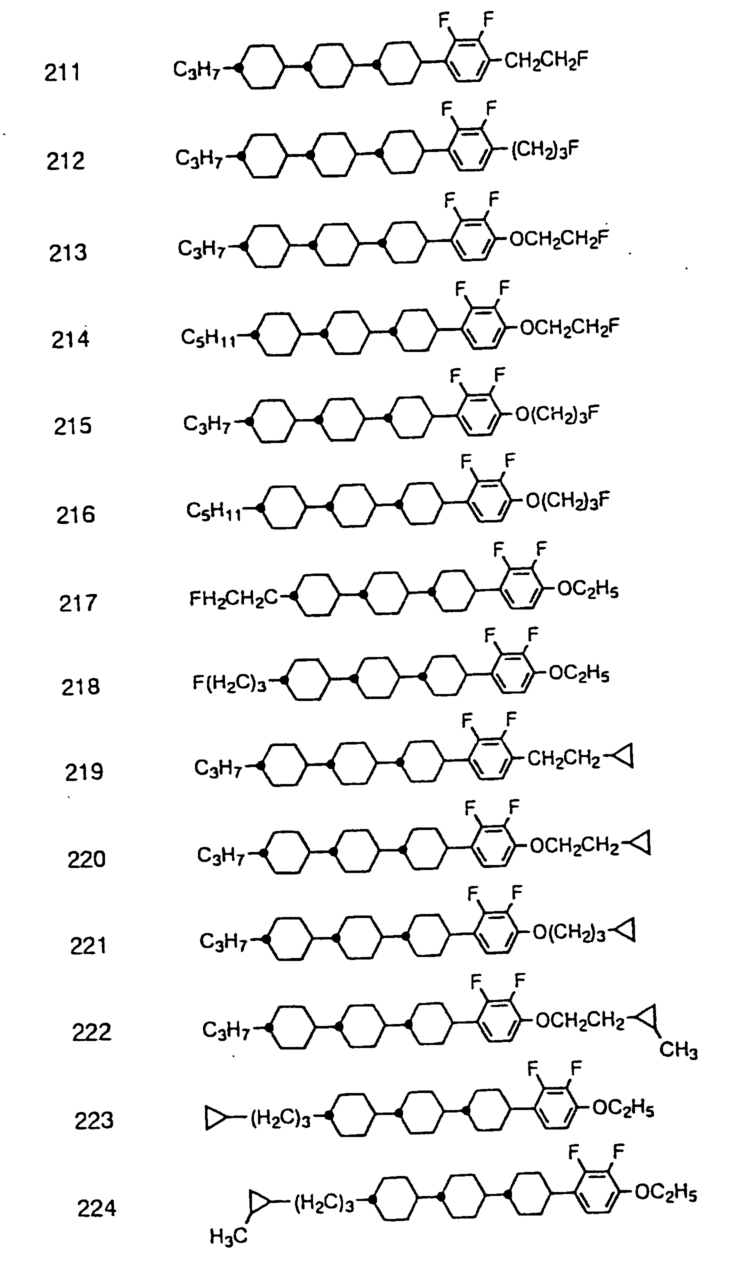

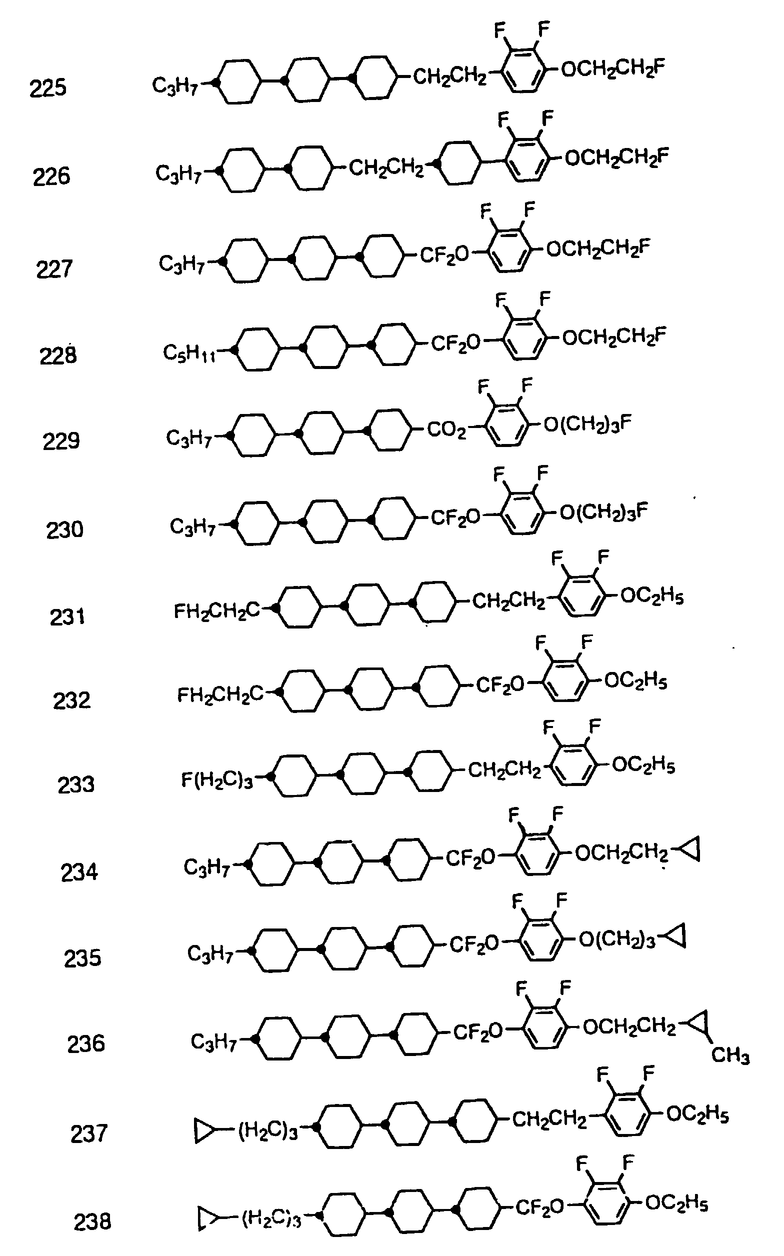

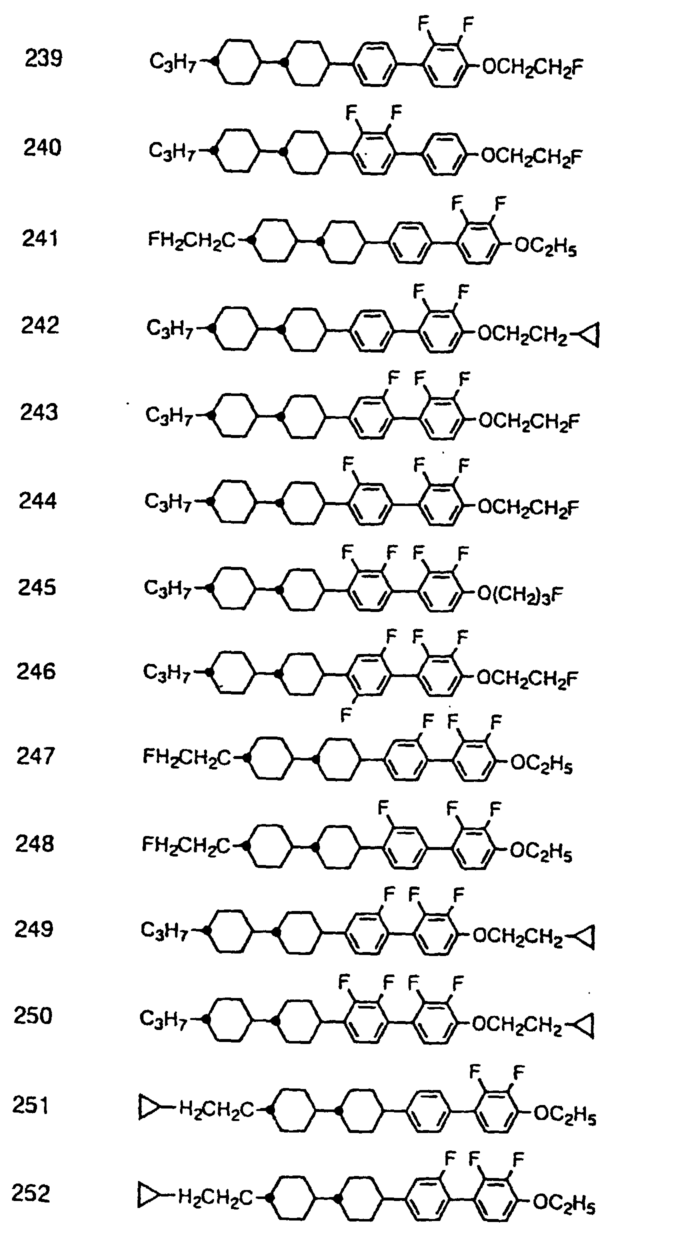

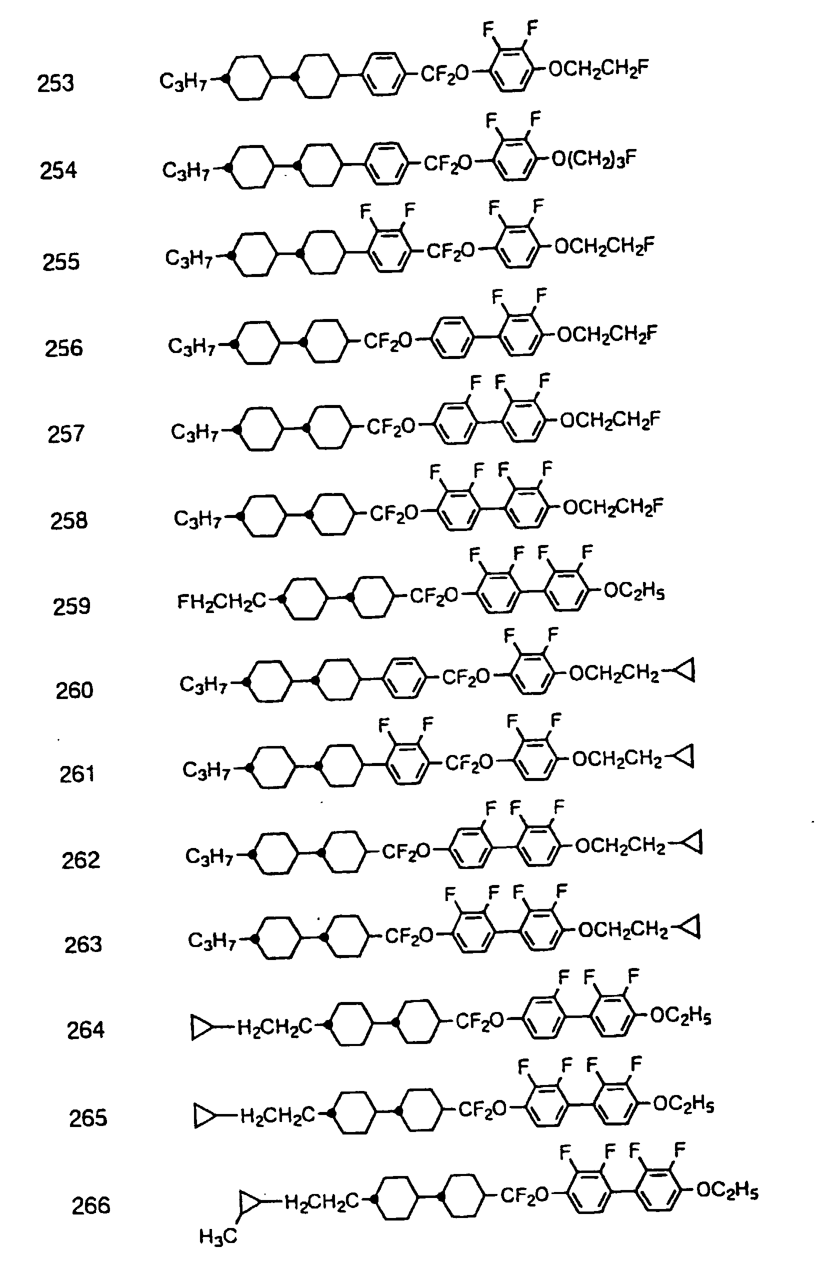

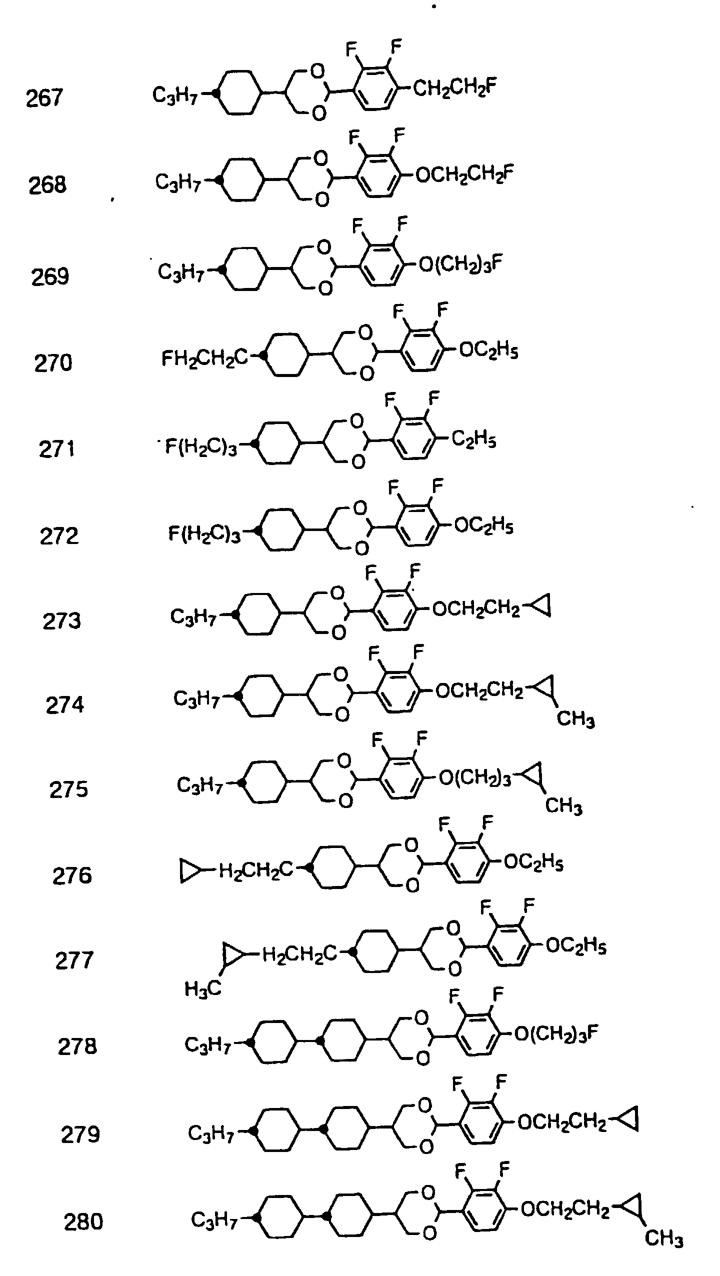

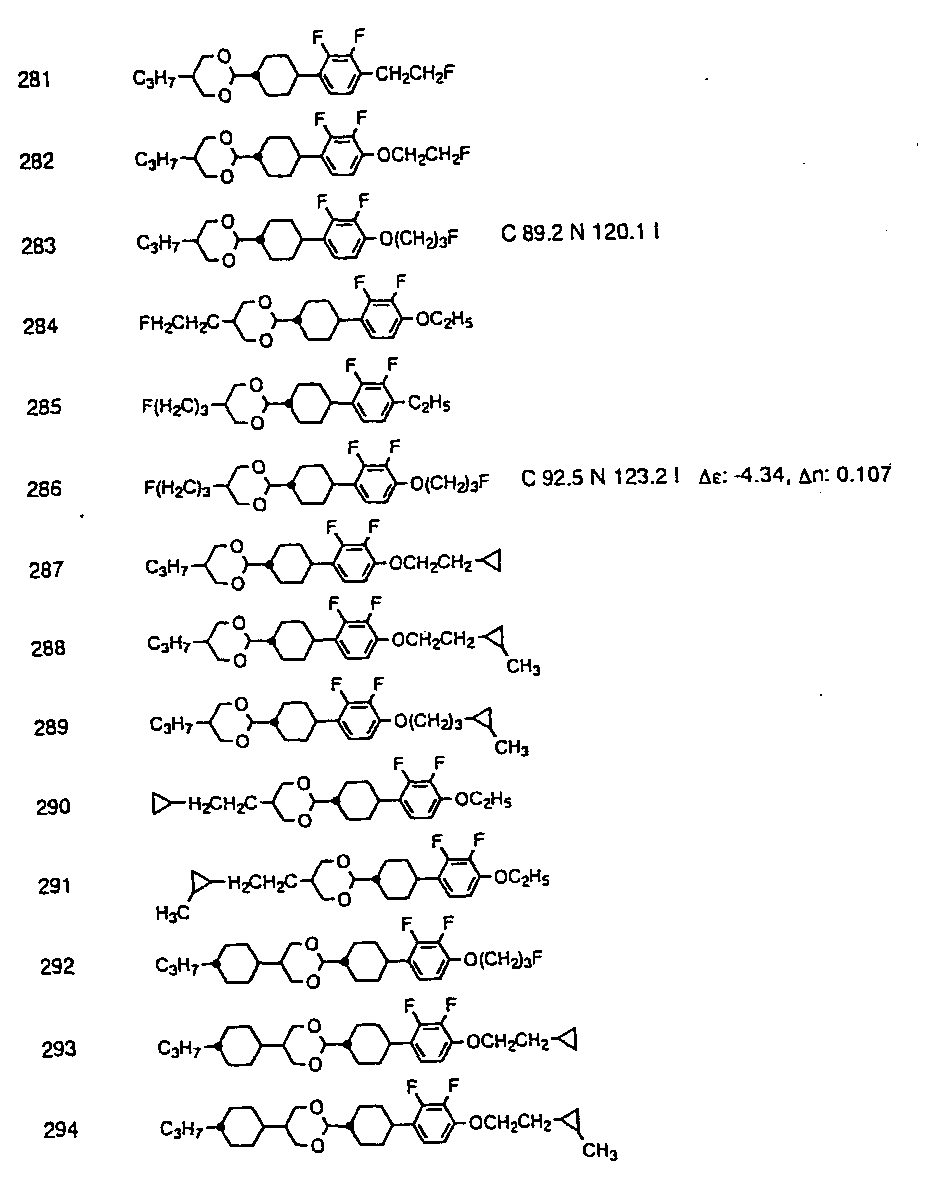

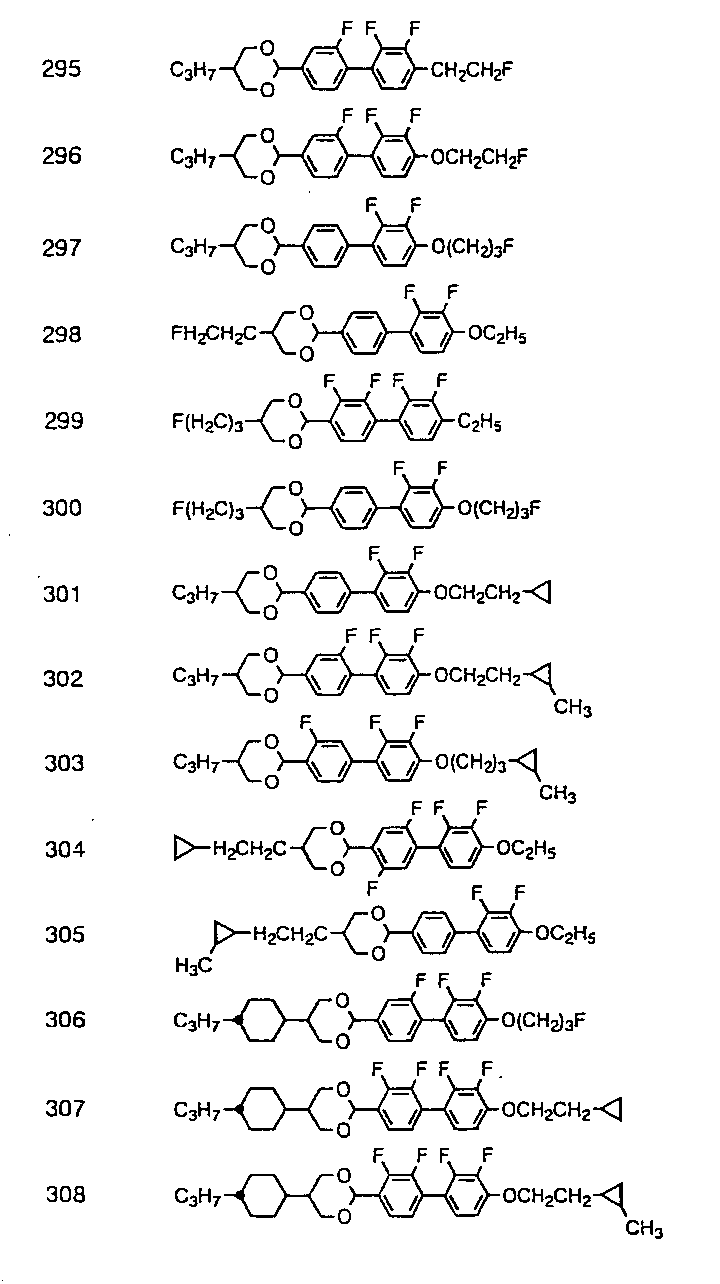

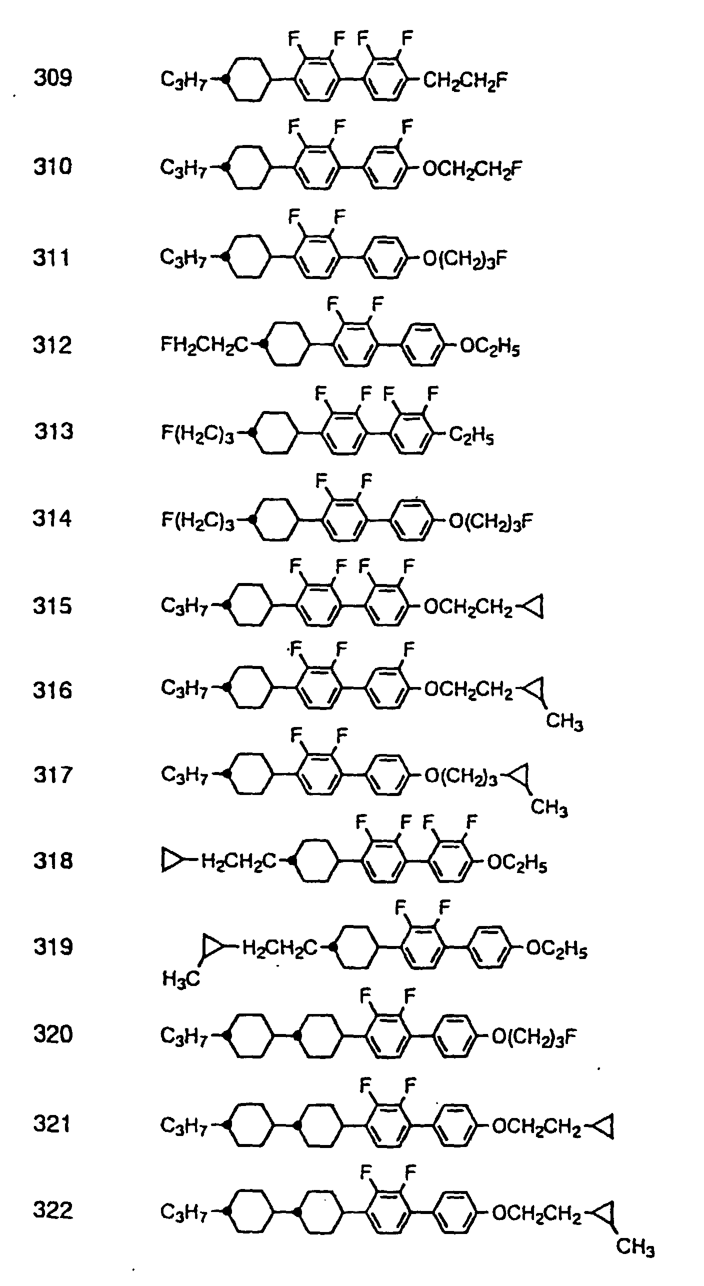

### Comparative Example 1

According to the method disclosed by V. Reiffenrath et al. described above, 2,3-difluoro-4-(4-(4-propylcyclohexyl) cyclohexyl)-1-ethoxybenzene (Compound No. 13) was prepared. Phase transition temperatures of this compound were as follows:
C 76.1 SB 79.9 N 184.9 I

Extrapolated values calculated from the liquid crystal composition obtained by mixing 15 % by weight of the compound with the composition (A) shown in Example 2 (Use Example 1) were as follows:
Δε = -4.40
Δn = 0.141

Extrapolated values of the 1-(4-propylcyclohexyl)-4-(2,3-difluoro-4-(3-fluoropropyl)phenyl)cyclohexane (15 %) shown in Example 2 were: Δε = -4.5 and Δn = 0.092, respectively. Thus, it was confirmed that the compounds of the present invention exhibit a large dielectric anisotropy value and a small optical anisotropy value at the same time compared with heretofore known compounds.

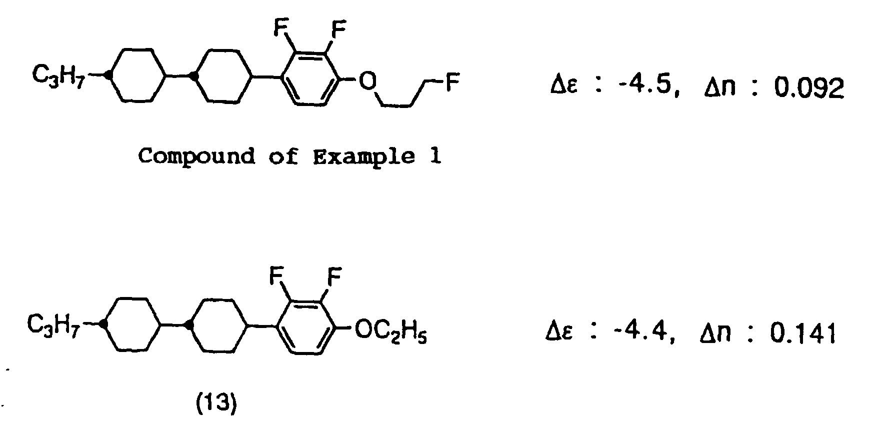

### Example 5 (Use Example 2 through Use Example 16)

Using various compounds expressed by the general formula (1), liquid crystal compositions shown in Use Example 2 through use Example 16 were prepared. In the following use examples, "%" means % by weight unless otherwise specified, and when cis-trans isomers exist in a particular compound, the compound used was trans-form. Further, the compounds in the following use examples are designated by symbols according to the definitions shown in the following Table 1 and Table 2. Viscosity (η) was determined at 20°C.

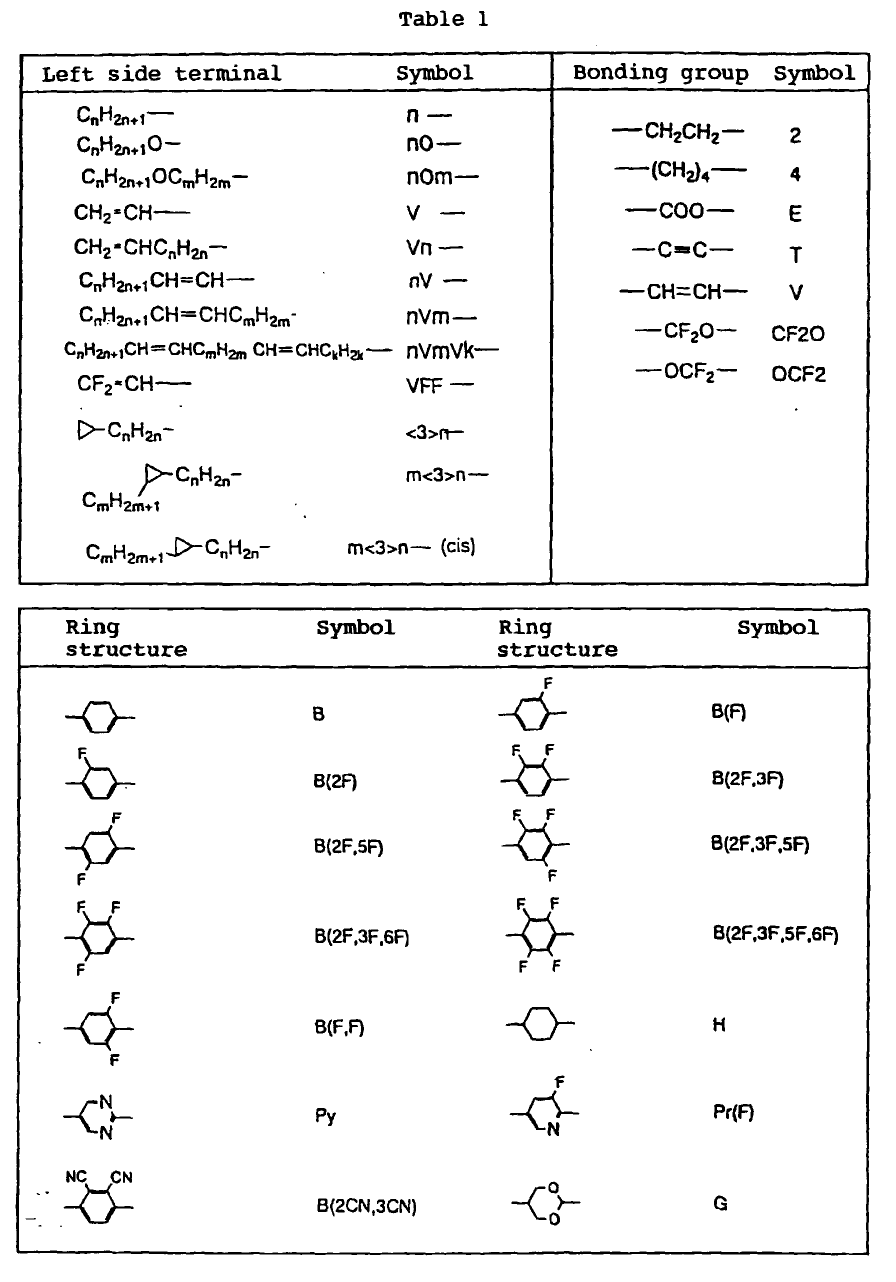

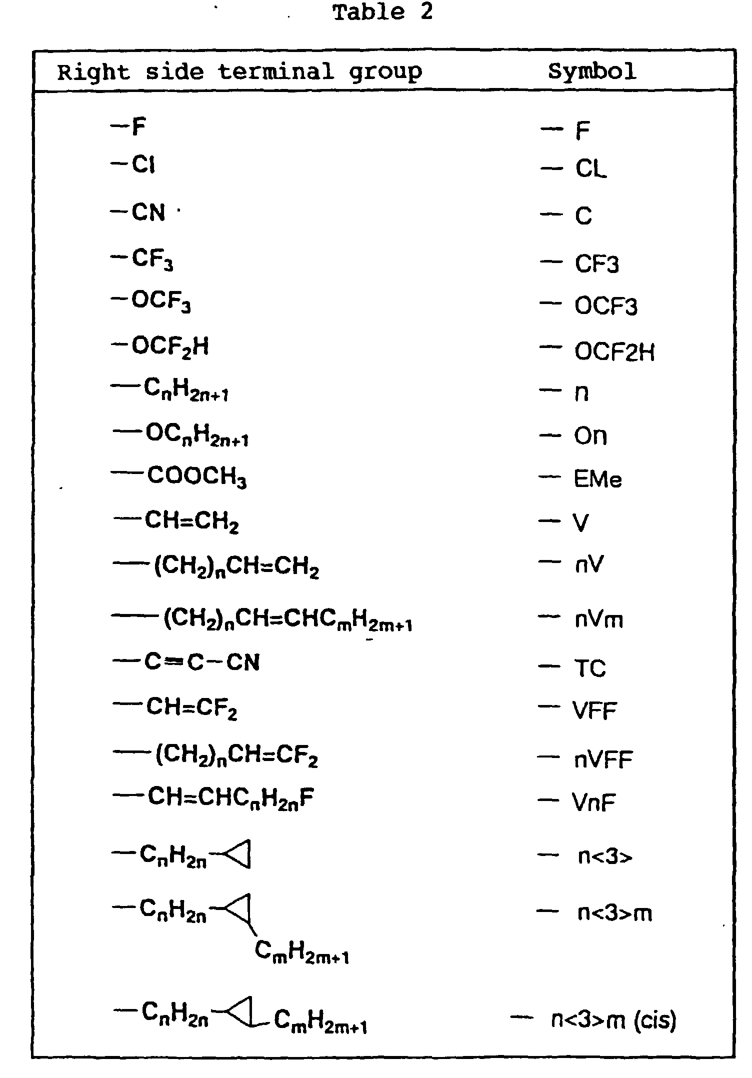

### Use Example 2

| | |
|---|---|
| 5 - HHB (2F, 3F) - O3F | 3. 0% |
| F2 - HHB (2F, 3F) - O2 | 3. 0% |
| 3 - HB (2F) B (2F, 3F) - O4F | 3. 0% |
| 3 - HB (2F, 3F) - O3F | 5. 0% |
| F3 - GHB (2F, 3F) - O2 〈3〉 | 2. 0% |
| 3 - HHB (2F, 3F) - O2 〈3〉 | 3. 0% |
| 〈3〉 2 - HHB (2F, 3F) - O2 | 3. 0% |
| 3 HB (2F) B (2F, 3F) - O2 〈3〉 | 3. 0% |
| 〈3〉 2O - HHB (2F, 3F) - O2 | 3. 0% |
| 4 - HEB - O2 | 20. 0% |
| 5 - HEB - O1 | 20. 0% |
| 3 - HEB - O2 | 18. 0% |
| 5 - HEB - O2 | 14. 0% |

T_{NI} = 82.1 (°C)
η = 23.9 (mPa·s)
Δn = 0.117
Δε = -2.1

### Use Example 3

| | |
|---|---|
| F2 - HHB (2F, 5F) - O2 | 14. 0% |
| 3 - HH - 2 | 5. 0% |
| 3 - HH - 4 | 6. 0% |
| 3 - HH - O1 | 4. 0% |
| 3 - HH - O3 | 5. 0% |
| 5 - HH - O1 | 4. 0% |
| 3 - HB (2F, 3F) - O2 | 12. 0% |
| 5 - HB (2F, 3F) - O2 | 11. 0% |
| 5 - HHB (2F, 3F) - O2 | 15. 0% |
| 3 - HHB (2F, 3F) - 2 | 24. 0% |

T_{NI} = 79.7 (°C)
Δn = 0.080
Δε = -4.1

### Use Example 4

| | |
|---|---|
| 〈3〉 2O - HHB (2F, 3F) - O2 | 12. 0% |
| 3 - HH - 5 | 5. 0% |
| 3 - HH - 4 | 5. 0% |
| 3 - HH - O1 | 6. 0% |
| 3 - HH - O3 | 6. 0% |
| 3 - HB - O1 | 5. 0% |
| 3 - HB - O2 | 5. 0% |
| 3 - HB (2F, 3F) - O2 | 10. 0% |
| 5 - HB (2F, 3F) - O2 | 10. 0% |
| 5 - HHB (2F, 3F) - O2 | 13. 0% |
| 3 - HHB (2F, 3F) - 2 | 4. 0% |
| 3 - HHB (2F, 3F) - 2 | 4. 0% |
| 3 - HHEH - 3 | 5. 0% |
| 3 -HHEH- 5 | 5. 0% |
| 4 - HHEH - 3 | 5. 0% |

T_{NI} = 79.8 (°C)
Δn = 0.078
Δε = -3.4

### Use Example 5

| | |
|---|---|
| 3 - HB (2F) B (2F, 3F) - O4 F | 10. 0% |
| 3 - HB (2F) B (2F, 3F) - O2 〈3〉 | 10. 0% |
| 3 - BB (2F, 3F) - O2 | 12. 0% |
| 3 - BB (2F, 3F) - O4 | 10. 0% |
| 5 - BB (2F, 3F) - O4 | 10. 0% |
| 2 - BB (2F, 3F) B - 3 | 5. 0% |
| 3 - BB (2F, 3F) B - 5 | 13. 0% |
| 5 - BB (2F, 3F) B - 5 | 14. 0% |
| 5 - BB (2F, 3F) B - 7 | 16. 0% |

T_{NI} = 74.7 (°C)
Δn = 0.174
Δε = -4.4

### Use Example 6

| | |
|---|---|
| F2 - HHB (2F, 3F) - O2 | 6. 0% |
| 3 - HB (2F) B (2F, 3F) - O4F | 6. 0% |
| 3 - HB (2F) B (2F, 3F) - O2 〈3〉 | 6. 0% |
| 〈3〉 2O - HHB (2F, 3F) - O2 | 6. 0% |
| 3 - BB (2F, 3F) - O2 | 10. 0% |
| 5 - BB - 5 | 9. 0% |
| 5 - BB - O6 | 9. 0% |
| 3 - BEB - 5 | 6. 0% |
| 3 - HEB - O2 | 13. 0% |
| 5 - BBB (2F, 3F) - 7 | 9. 0% |
| 3 - H2BB (2F) - 5 | 20. 0% |

T_{NI} = 94.6 (°C)
Δn = 0.130
Δε = -4.2

### Use Example 7

| | |
|---|---|
| 〈3〉 2O - HHB (2F, 3F) - O2 | 6. 0% |
| 3 - HHB (2F, 3F) - O2 〈3〉 1 | 3. 0% |
| 3 - HHB (2F, 3F) - O3 〈3〉 1 | 3. 0% |
| 3 - HB - O1 | 15. 0% |
| 3 - HB - O2 | 6. 0% |
| 3 - HEB (2F, 3F) - O2 | 9. 0% |
| 4 - HEB (2F, 3F) - O2 | 9. 0% |
| 5 - HEB (2F, 3F) - O2 | 9. 0% |
| 2 - BB2B - O2 | 6. 0% |
| 3 - BB2B - O2 | 6. 0% |
| 1 - B2BB (2F) - 5 | 7. 0% |
| 3 - B2BB (2F) - 5 | 7. 0% |
| 3 - BB (2F, 3F) B - 3 | 7. 0% |

T_{NI} = 81.2 (°C)
Δn = 0.156

### Use Example 8

| | |
|---|---|
| 3 - HHB (2F, 3F) - O2 〈3〉 | 5. 0% |
| 〈3〉 2 - HHB (2F, 3F) - O2 | 5. 0% |
| 〈3〉 3 - HHB (2F, 3F) - O2 | 4. 0% |
| 〈3〉 2V - HHB (2F, 3F) - O2 | 3. 0% |
| 3 - HB - O1 | 9. 0% |
| 3 - HB - O2 | 9. 0% |
| 3 - HB - O4 | 9. 0% |
| 2 - BTB - O1 | 5. 0% |
| 2 - BTB - O1 | 5. 0% |
| 1 - BTB - O2 | 5. 0% |
| 3 - BTB (2F, 3F) - O2 | 13. 0% |
| 3 - B (2F, 3F) TB (2F, 3F) - O4 | 4. 0% |
| 3 - HBTB - O1 | 5. 0% |
| 3 - HBTB - O2 | 5. 0% |
| 3 - HBTB - O3 | 5. 0% |
| 3 - HHB (2F, 3F) - O2 | 6. 0% |
| 5 - HBB (2F, 3F) - O2 | 5. 0% |
| 5 - BPr (F) - O2 | 3. 0% |

T_{NI} = 79.2 (°C)
Δn = 0.201

### Use Example 9

| | |
|---|---|
| F2 - HHB (2F, 3F) - O2 | 8. 0% |
| F3 - GHB (2F, 3F) - O3F | 5. 0% |
| 〈3〉 2O - HHB (2F, 3F) - O2 | 9. 0% |
| 3 - HB - O2 | 10. 0% |
| 5 - HB - 3 | 8. 0% |
| 5 - BR (2F, 3F) - O2 | 10. 0% |
| 5 - HB (2F, 3F) - O2 | 8. 0% |
| 5 - HHB (2F, 3F) - O2 | 4. 0% |
| 5 - HHB (2F, 3F) - 1O1 | 4. 0% |
| 2 - HHB(2F, 3F) - 1 | 5. 0% |
| 3 - HHB (2F, 3F) -1 | 5. 0% |
| 3 - HBB - 2 | 6. 0% |
| 3 - BB (2F, 3F) B - 3 | 8. 0% |
| 5 - B2BB (2F, 3F) - O2 | 10. 0% |

T_{NI} = 77.6 (C)
Δn = 0.130
Δε = -4.0

### Use Example 10

| | |
|---|---|
| 5 - HHB (2F, 3F) - O3F | 3. 0% |
| 3 - HB (2F) B (2F, 3F) - O4F | 3. 0% |
| F3 - GHB (2F, 5F) - O3F | 3. 0% |
| 3 - H2B (2F) B (2F, 3F) - O2 〈3〉 | 3. 0% |
| 〈3〉 2O - HHB (2F, 3F) - O2 | 3. 0% |
| 3 - HB - O2 | 20. 0% |
| 1O1 - HH - 3 | 6. 0% |
| 3 - HH - EMe | 12. 0% |
| 4 - HEB - O1 | 9. 0% |
| 4 - HEB - O2 | 7. 0% |
| 5 - HEB - O1 | 8. 0% |
| 3 - HHB - 1 | 6. 0% |
| 3 - HHB - 3 | 6. 0% |
| 3 - HEB (2CN, 3CN)- O5 | 4. 0% |
| 4 - HEB (2CN, 3CN) -O5 | 3. 0% |
| 5 - HEB (2CN, 3CN) - O5 | 2. 0% |
| 2 - HBEB (2CN, 3CN) - O2 | 2. 0% |

T_{NI} = 82.3 (°C)
η = 29.0 (mPa·s)
Δn = 0.085
Δε = -3.6

### Use Example 11

| | |
|---|---|
| 3 - HB (2F) B (2F, 3F) - O4F | 4. 0% |
| 3 - HB (2F) B (2F, 3F) - O2〈3〉 | 3. 0% |
| 〈3〉 2V - HHB (2F, 3F) - O2 | 2. 0% |
| 1V2 - BEB (F, F) - C | 5. 0% |
| 3 -HB- C | 20. 0% |
| V2 -HB- C | 6. 0% |
| 1 - BTB - 3 | 5. 0% |
| 2 - BTB - 1 | 10. 0% |
| 1O1 - HH - 3 | 3. 0% |
| 3 - HH - 4 | 11. 0% |
| 3 -HHB- 1 | 11. 0% |
| 3 -HHB- 3 | 3. 0% |
| 3 -H2BTB- 2 | 4. 0% |
| 3 - H2BTB- 3 | 4. 0% |
| 3 -HB(F)TB- 2 | 6. 0% |
| 3 -HHB- C | 3. 0% |

T_{NI} = 84.4 (°C)
η = 18.1 (mPa·s)
Δn = 0.154
Δε = 6.6
Vth = 2.31

### Use Example 12

| | |
|---|---|
| 〈3〉2O -HHB (2F ,3F)- O2 | 10. 0% |
| 5- PyB- F | 4. 0% |
| 3- PyB(F)- F | 4. 0% |
| 2 -BB- C | 5. 0% |
| 4 -BB- C | 4. 0% |
| 5 -BB- C | 5. 0% |
| 2 -PyB- 2 | 2. 0% |
| 3 -PyB- 2 | 2. 0% |
| 4 -PyB- 2 | 2. 0% |
| 6 -PyB- O5 | 3. 0% |
| 6 -PyB- O6 | 3. 0% |
| 6 -PyB- O7 | 3.0% |
| 6 -PyB- O8 | 3. 0% |
| 3 -PyBB- F | 6. 0% |
| 4 -PyBB- F | 6. 0% |
| 5 -PyBB- F | 6. 0% |
| 3 -HHB- 1 | 6. 0% |
| 3 -HHB- 3 | 8. 0% |
| 2 -H2BTB- 2 | 4. 0% |
| 2 -H2BTB- 3 | 4. 0% |
| 3 -H2BTB- 2 | 5. 0% |
| 3 -H2BTB- 3 | 5. 0% |

T_{NI} = 90.6 (°C)
η = 37.8 (mPa·s)
Δn = 0.190
Δε = 5.8
Vth = 2.56

### Use Example 13

| | |
|---|---|
| 5 -HHB (2F,3F)- O3F | 3. 0% |
| F2 -HHB(2F,5F)- O2 | 3. 0% |
| 3 -HB (2F,3F)- O3F | 2. 0% |
| 〈3〉 3 -HHB(2F,3F)- O2 | 2. 0% |
| 2O1 -BEB(F)- C | 5. 0% |
| 3O1 -BEB(F)- C | 12. 0% |
| 1V2 BEB(F,F)- C | 10. 0% |
| 3 -HEB- O4 | 4. 0% |
| 3 -HH- EMe | 6. 0% |
| 3 -HB- O2 | 18. 0% |
| 7 -HEB- F | 2. 0% |
| 3 -HHEB- F | 2. 0% |
| 5 -HHEB- F | 2. 0% |
| 3 -HBEB- F | 4. 0% |
| 2O1 -HBEB(F)- C | 2. 0% |
| 3 -HB(F)EB(F)- C | 2. 0% |
| 3 -HBEB(F,F)- C | 2. 0% |
| 3 -HHB- F | 4. 0% |
| 3 -HHB- O1 | 4. 0% |
| 3 -HHB- 3 | 3. 0% |
| 3 -HEBEB- F | 2. 0% |
| 3 -HEBEB- 1 | 2. 0% |
| 3 -HHB(F)- C | 4. 0% |

T_{NI} = 74.0 (°C)
η = 36.2 (mPa·s)
Δn = 0.113
Δε = 22.1
Vth = 1.06

### Use Example 14

| | |
|---|---|
| 3 -HB(2F)B(2F, 3F)- O4F | 4. 0% |
| F3 -GHB(2F, 3F)- O3F | 3. 0% |
| 3 -HB(2F)B(2F, 3F)- O2〈3〉 | 3. 0% |
| 1V2 -BEB(F,F)- C | 6. 0% |
| 3 -HB- C | 10. 0% |
| 2 -BTB- 1 | 10. 0% |
| 5 -HH- VFF | 20. 0% |
| 1 -BHH- VFF | 8. 0% |
| 1 -BHH- 2VFF | 11. 0% |
| 3 -H2BTB- 2 | 5.0% |
| 3 -H2BTB- 3 | 4. 0% |
| 3 -H2BTB- 4 | 4. 0% |
| 3 -HHB- 1 | 4. 0% |

T_{NI} = 87.6 (°C)
η = 18.6 (mPa·s)
Δn = 0.134
Δε = 6.0
Vth = 2.29

### Use Example 15

| | |
|---|---|
| F2 -HHB(2F,3F)- O2 | 5. 0% |
| 〈3〉2O -HHB(2F,3F)- O2 | 5. 0% |
| 5 -HB- F | 12. 0% |
| 6 -HB- F | 9. 0% |
| 7 -HB- F | 7. 0% |
| 2 -HHB- OCF3 | 7. 0% |
| 3 -HHB- OCF3 | 7. 0% |
| 4 -HHB- OCF3 | 7. 0% |
| 5 -HHB- OCF3 | 5. 0% |
| 3 -HH2B- OCF3 | 4. 0% |
| 3 -HH2B- OCF3 | 4. 0% |
| 3 -HHB(F, F)- OCF3 | 5. 0% |
| 3 -HBB(F)- F | 10. 0% |
| 3 -HH2B(F)- F | 3. 0% |
| 3 -HB(F)BH- 3 | 3. 0% |
| 5 -HBBH- 3 | 3. 0% |
| 3 -HHB(F, F)- OCF2H | 4. 0% |

T_{NI} = 89.3 (°C)
η = 18.6 (mPa·s)
Δn = 0.090
Δε = 3.2
Vth = 2.76

### Use Example 16

| | |
|---|---|
| F3 -GHB(2F, 3F)- O3F | 2. 0% |
| 〈3〉 2 -HHB (2F, 3F)- O2 | 2. 0% |
| 〈3〉 2V -HHB(2F, 3F)- O2 | 2. 0% |
| 3 -HHB(2F, 3F)- O2〈3〉1 | 2. 0% |
| 3 -HHB(2F, 3F)- O3〈3〉1 | 2. 0% |
| 7 -HB(F, F)- F | 5. 0% |
| 3 -H2HB(F, F)- F | 12. 0% |
| 4 -H2HB(F,F)- F | 6. 0% |
| 3 -HHB(F,F)- F | 10. 0% |
| 3 -HBB(F,F)- F | 10. 0% |
| 3 -HHEB (F,F)- F | 10. 0% |
| 4 -HHEB(F,F)- F | 3. 0% |
| 5 -HHEB(F,F)- F | 3. 0% |
| 3 -HGB(F,F)- F | 15. 0% |
| 3 -HBCF2OB- OCF3 | 4. 0% |
| 3 -HHBB(F,F)- F | 6. 0% |

T_{NI} = 79.2 (°C)
Δn = 0.092
Δε = 10.1

Liquid crystalline compounds of the present invention have a negative and extremely large dielectric anisotropy value and a small optical anisotropy value at the same time. Accordingly, liquid crystal compositions exhibiting a low threshold voltage and a small optical anisotropy value can be actualized by using the liquid crystalline compound of the present invention as component of liquid crystal compositions. Further, excellent liquid crystal display devices can be provided by using the composition.

Excellent characteristics of the liquid crystalline compounds of the present invention described herein contradict to the existing concept (theory of Maier and Meier) described above. Liquid crystalline compounds having such excellent characteristics have not been known at all up to now.

Liquid crystalline compounds of the present invention are sufficiently stable physically and chemically under conditions in which liquid crystal display devices are ordinarily used, and are considerably excellent as component of nematic liquid crystal compositions. For instance, the compound of Example 1 exhibits an extremely high chemical stability compared with heretofore known cyan compounds (for example, Compound No. 14).

### INDUSTRIAL APPLICABILITY

Liquid crystalline compounds of the present invention can preferably be used for liquid crystal compositions for IPS mode or VA mode since the compounds have a very excellent miscibility with other liquid crystal materials even at low temperatures. Also, they can be utilized not only for IPS and VA modes, but also for ECB (electrically controlled birefringence) mode and GH (guest-host) mode. Further, the compounds can preferably be employed as liquid crystal material for TN (twisted nematic), STN (super twisted nematic), or AM (active matrix) mode.

## Claims

1. A 2,3-difluorophenyl derivative expressed by the general formula (1) 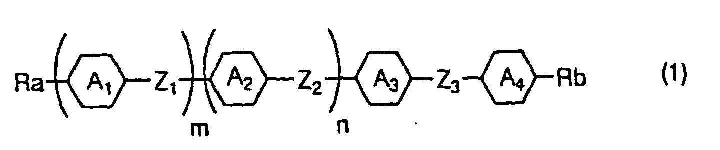 wherein Ra and Rb each independently represent a straight chain or branched alkyl group having 1 to 10 carbon atoms or a straight chain or branched alkoxy group having 1 to 10 carbon atoms in which groups any methylene group may be replaced by -O-, -CH=CH-, or C≡C-, but there is not a case wherein -O-continues, provided that any one or more methylene groups in at least one of the Ra and Rb are replaced by cyclopropane-1,2-diyl, -CF₂-, or -CFH-; ring A₁ to ring A₄ each independently represent cyclohexane-1,4-diyl or 1,4-phenylene, but hydrogen atom on these rings may be replaced by a halogen atom and not-adjacent any methylene group in the cyclohexane ring may be replaced by -O-, provided that at least one of the ring A₃ and ring A₄ represents 2,3-difluoro-1,4-phenylene; Z₁, Z₂, and Z₃ each independently represent single bond, -(CH₂)ₚ-, -CO₂-, -CF₂O-, or -CH₂O- provided that p is an integer of 2 to 4; m and n are each independently 0 or 1; and any atom which constitutes the compound may be replaced by its isotope.

2. The 2,3-difluorophenyl derivative according to claim 1 wherein ring A₃ in the general formula (1) is 2,3-difluorophenylene.

3. The 2,3-difluorophenyl derivative according to claim 1 wherein ring A₄ in the general formula (1) is 2,3-difluorophenylene.

4. The 2,3-difluorophenyl derivative according to claim 2 wherein at least any one of the Ra and Rb in the general formula (1) is ω-fluoroalkyl group or ω-fluoroalkoxy group having 3 to 10 carbon atoms.

5. The 2,3-difluorophenyl derivative according to claim 3 wherein at least any one of the Ra and Rb in the general formula (1) is ω-fluoroalkyl group or ω-fluoroalkoxy group having 3 to 10 carbon atoms.

6. The 2,3-difluorophenyl derivative according to claim 2 wherein at least any one of the Ra and Rb in the general formula (1) is an alkyl group or alkoxy group having cyclopropyl group at its terminal.

7. The 2,3-difluorophenyl derivative according to claim 3 wherein at least any one of the Ra and Rb in the general formula (1) is an alkyl group or alkoxy group having cyclopropyl group at its terminal.

8. The 2,3-difluorophenyl derivative according to claim 2 wherein at least any one of the Ra and Rb in the general formula (1) is an alkyl group or alkoxy group having 2-methylcyclopropyl group at its terminal.

9. The 2,3-difluorophenyl derivative according to claim 3 wherein at least any one of the Ra and Rb in the general formula (1) is an alkyl group or alkoxy group having 2-methylcyclopropyl group at its terminal.

10. A liquid crystal composition comprising at least one liquid crystalline compound defined in claim 1.

11. A liquid crystal composition comprising, as a first component, at least one liquid crystalline compound defined in claim 1 and comprising, as a second component, at least one compound selected from the group consisting of the compounds expressed by any one of the general formulas (2), (3), and (4) 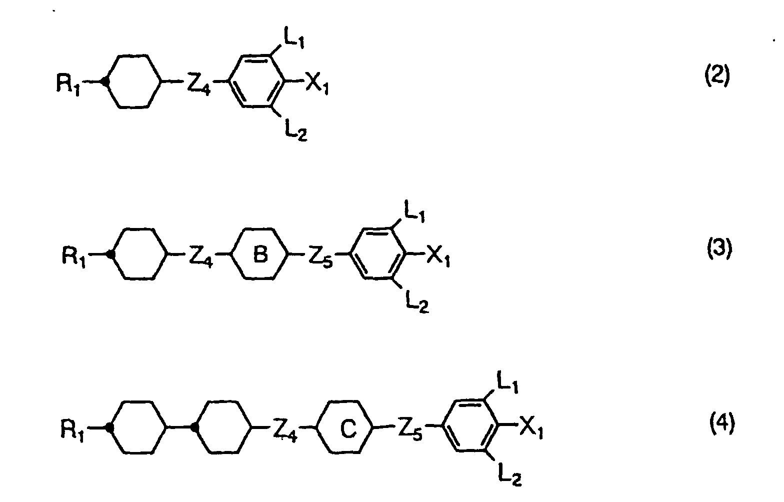 wherein R₁ represents an alkyl group having 1 to 10 carbon atoms in which group not-adjacent any methylene group may be replaced by -O- or -CH=CH-, and any hydrogen atom in the group may be replaced by fluorine atom; X₁ represents fluorine atom, chlorine atom, -OCF₃, -OCF₂H, -CF₃, -CF₂H, -CFH₂, -OCF₂CF₂H, or -OCF₂CFHCF₃; L₁ and L₂ each independently represent hydrogen atom or fluorine atom; Z₃ and Z₄ each independently represent -CH₂CH₂-, -(CH₂)₄-, -COO-, -CF₂O-, -OCF₂-, -CH=CH-, or single bond; ring B represents cyclohexane-1,4-diyl or 1,3-dioxane-2,5-diyl, or 1,4-phenylene in which hydrogen atom may be replaced by fluorine atom; ring C represents cyclohexane-1,4-diyl, or 1,4-phenylene in which hydrogen atom may be replaced by fluorine atom; and any atom which constitutes the compounds may be replaced by its isotope.

12. A liquid crystal composition comprising, as a first component, at least one liquid crystalline compound defined in claim 1 and comprising, as a second component, at least one compound selected from the group consisting of the compounds expressed by the general formula (5) or (6) 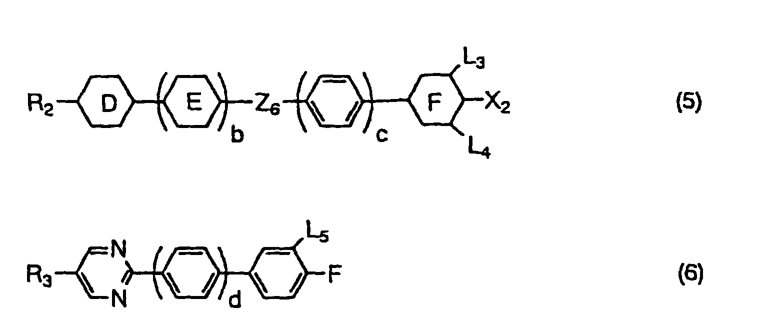 wherein R₂ and R₃ each independently represent an alkyl group having 1 to 10 carbon atoms in which group not-adjacent methylene group may be replaced by -O- or -CH=CH- and any hydrogen atom in the group may be replaced by fluorine atom; X₂ represents -CN or -C≡C-CN; ring D represents cyclohexane-1,4-diyl, 1,4-phenylene, 1,3-dioxane-2,5-diyl, or pyrimidine-2,5-diyl; ring E represents cyclohexane-1,4-diyl, 1,4-phenylene in which hydrogen atom may be replaced by fluorine atom, or pyrimidine-2,5-diyl; ring F represents cyclohexane-1,4-diyl or 1,4-phenylene; Z₅ represents -CH₂CH₂-, -COO-, or single bond; L₃, L₄, and L₅ each independently represent hydrogen atom or fluorine atom; b, c, and d are each independently 0 or 1; and any atom which constitutes the compounds may be replaced by its isotope.

13. A liquid crystal composition comprising, as a first component, at least one liquid crystalline compound defined in claim 1 and comprising, as a second component, at least one compound selected from the group consisting of the compounds expressed by any one of the general formulas (7), (8), and (9) 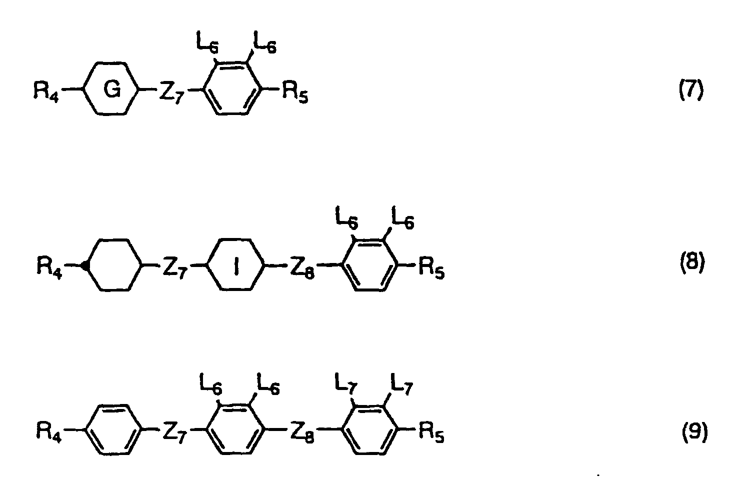 wherein R₄ and R₅ each independently represent an alkyl group having 1 to 10 carbon atoms in which group not-adjacent any methylene group may be replaced by -O- or -CH=CH- and any hydrogen atom in the group may be replaced by fluorine atom; ring G and ring I each independently represent cyclohexane-1,4-diyl or 1,4-phenylene; L₆ and L₇ each independently represent hydrogen atom, cyano group, or fluorine atom, but there is not a case wherein L₆ and L₇ represent hydrogen atom at the same time; Z₆ and Z₇ each independently represent -CH₂CH₂-, -COO-, or single bond; and any atom which constitutes the compounds may be replaced by its isotope.

14. A liquid crystal composition comprising, as a first component, at least one liquid crystalline compound defined in claim 1, comprising, as a second component, at least one compound selected from the group consisting of the compounds expressed by any one of the general formulas (2), (3), and (4) described above, and comprising, as a third component, at least one compound selected from the group consisting of the compounds expressed by any one of the general formulas (10), (11), and (12) 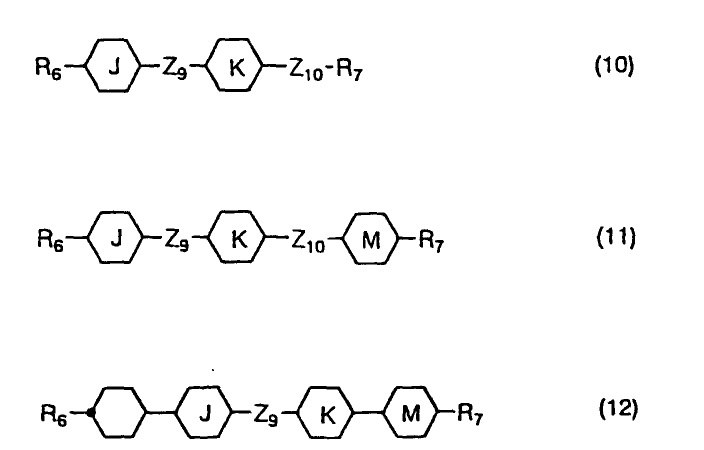 wherein R₆ and R₇ each independently represent an alkyl group having 1 to 10 carbon atoms in which group not-adjacent any methylene group may be replaced by -O- or -CH=CH- and any hydrogen atom in the group may be replaced by fluorine atom; ring J, ring K, and ring M each independently represent cyclohexane-1,4-diyl or pyrimidine-2,5-diyl, or 1,4-phenylene in which hydrogen atom may be replaced by fluorine atom; Z₈ and Z₉ each independently represent -CH₂CH₂-, -C≡C-, -COO-, -CH=CH-, or single bond; and any atom which constitutes the compounds may be replaced by its isotope.

15. A liquid crystal composition comprising, as a first component, at least one liquid crystalline compound defined in claim 1, comprising, as a second component, at least one compound selected from the group consisting of the compounds expressed by the general formula (5) or (6) described above, and comprising, as a third component, at least one compound selected from the group consisting of the compounds expressed by any one of the general formulas (10), (11), and (12) described above.

16. A liquid crystal composition comprising, as a first component, at least one liquid crystalline compound defined in claim 1, comprising, as a second component, at least one compound selected from the group consisting of the compounds expressed by any one of the general formulas (7), (8), and (9) described above, and comprising, as a third component, at least one compound selected from the group consisting of the compounds expressed by any one of the general formulas (10), (11), and (12) described above.

17. A liquid crystal composition comprising, as a first component, at least one liquid crystalline compound defined in claim 1, comprising, as a second component, at least one compound selected from the group consisting of the compounds expressed by any one of the general formulas (2), (3), and (4) described above, comprising, as a third component, at least one compound selected from the group consisting of the compounds expressed by the general formula (5) or (6) described above, and comprising, as a fourth component, at least one compound selected from the group consisting of the compounds expressed by any one of the general formulas (10), (11), and (12) described above.

18. A liquid crystal composition further comprising one or more optically active compounds in addition to the liquid crystal composition defined in any one of claims 10 to 17.

19. A liquid crystal display device fabricated by using a liquid crystal composition defined in any one of claims 10 to 18.
